(19) Europäisches Patentamt
European Patent Office
Office européen des brevets

(11) **EP 4 657 049 A1**

(12) **EUROPEAN PATENT APPLICATION**
published in accordance with Art. 153(4) EPC

(43) Date of publication:
**03.12.2025 Bulletin 2025/49**

(21) Application number: **24747109.7**

(22) Date of filing: **11.01.2024**

(51) International Patent Classification (IPC):
**G01N 23/18** (2018.01)   **G01N 23/04** (2018.01)
**G01N 23/046** (2018.01)   **G01N 23/087** (2018.01)

(52) Cooperative Patent Classification (CPC):
**G01N 23/04; G01N 23/046; G01N 23/087; G01N 23/18**

(86) International application number:
**PCT/JP2024/000432**

(87) International publication number:
**WO 2024/157778 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.01.2023 JP 2023008365**

(71) Applicant: **FUJIFILM Corporation
Tokyo 106-8620 (JP)**

(72) Inventor: **KATSUYAMA, Kimito
Tokyo 106-8620 (JP)**

(74) Representative: **Meissner Bolte Partnerschaft mbB
Patentanwälte Rechtsanwälte
Postfach 86 06 24
81633 München (DE)**

(54) **INFORMATION PROCESSING DEVICE, INFORMATION PROCESSING METHOD, AND INFORMATION PROCESSING PROGRAM**

(57) An information processing apparatus acquires projection data corresponding to each of a plurality of imaging positions at which an inspection target is irradiated with radiation from different directions, such that one piece of projection data is acquired at each of at least two imaging positions among the plurality of imaging positions, and generates a tomographic image of the inspection target, based on the projection data and attenuation information of the inspection target.

FIG. 7

EP 4 657 049 A1

## Description

Technical Field

The present disclosure relates to an information processing apparatus, an information processing method, and an information processing program.

Background Art

One known nondestructive inspection technique for detecting a defect in an inspection target such as a machine part or a structure without destroying the inspection target is a technique for generating a tomographic image of the inspection target from projection data obtained by irradiating the inspection target with radiation such as X-rays. For example, JP2014-61274A describes a technique for separating projection data into pieces of line-integrated data each corresponding to a respective one of a plurality of at least two or more preset reference materials and generating tomographic images of the respective reference materials from the pieces of line-integrated data of the respective reference materials.

SUMMARY OF THE INVENTION

Technical Problem

In nondestructive inspection using radiation, it is desirable to inspect an inspection target for defects at high speed, and it is desirable to generate a tomographic image even from a small number of pieces of projection data by reducing the number of times of imaging of the inspection target. In addition, the irradiation direction of radiation, that is, the projection direction, may be limited depending on the inspection target, and it is desirable to generate a tomographic image even from projection data where the projection direction is limited.

The present disclosure provides an information processing apparatus, an information processing method, and an information processing program that enable generation of a high-accuracy tomographic image even from a small number of pieces of projection data or projection data with limited projection directions.

Solution to Problem

A first aspect of the present disclosure provides an information processing apparatus including at least one processor configured to acquire projection data corresponding to each of a plurality of imaging positions at which an inspection target is irradiated with radiation from different directions, such that one piece of projection data is acquired at each of at least two imaging positions among the plurality of imaging positions, and generate a tomographic image of the inspection target, based on the projection data and attenuation information of the inspection target.

An information processing apparatus according to a second aspect of the present disclosure may be the information processing apparatus according to the first aspect, in which the attenuation information may include attenuation information corresponding to each of at least two members included in inspection target.

An information processing apparatus according to a third aspect of the present disclosure may be the information processing apparatus according to the first aspect, in which the attenuation information may include attenuation information of air.

An information processing apparatus according to a fourth aspect of the present disclosure may be the information processing apparatus according to the first aspect, in which the attenuation information may include attenuation information for at least two energy bands.

An information processing apparatus according to a fifth aspect of the present disclosure may be the information processing apparatus according to the first aspect, in which the processor may be configured to generate the tomographic image, based on scattered radiation produced by the inspection target.

An information processing apparatus according to a sixth aspect of the present disclosure may be the information processing apparatus according to the first aspect, in which the processor may be configured to generate the tomographic image, based on a length of a path along which the radiation is transmitted through the inspection target.

In an information processing apparatus according to a seventh aspect of the present disclosure, the radiation may be emitted as any one of a parallel beam, a fan beam, and a cone beam.

An eighth aspect of the present disclosure provides an information processing method including a process performed by a processor, the process including acquiring projection data corresponding to each of a plurality of imaging positions at which an inspection target is irradiated with radiation from different directions, such that one piece of projection data is acquired at each of at least two imaging positions among the plurality of imaging positions; and generating a tomographic image of the inspection target, based on the projection data and attenuation information of the inspection target.

A ninth aspect of the present disclosure provides an information processing program for causing a processor to perform a process including acquiring projection data corresponding to each of a plurality of imaging positions at which an inspection target is irradiated with radiation from different directions, such that one piece of projection data is acquired at each of at least two imaging positions among the plurality of imaging positions; and generating a tomographic image of the inspection target, based on the projection data and attenuation information of the inspection target.

Advantageous Effects of Invention

According to the aspects described above, the information processing apparatus, the information processing method, and the information processing program of the present disclosure enable generation of a high-accuracy tomographic image even from a small number of pieces of projection data or projection data with limited projection directions.

BRIEF DESCRIPTION OF THE DRAWINGS

Fig. 1 is a block diagram illustrating an example overall configuration of a tomographic image generation system according to an exemplary embodiment;
Fig. 2 is a block diagram illustrating an example configuration of an information processing apparatus;
Fig. 3 is a functional block diagram illustrating an example configuration of the information processing apparatus;
Fig. 4 is a flowchart illustrating an example of a table generation process executed by the information processing apparatus;
Fig. 5A is a diagram illustrating the setting of paths of radiation from a radiation source to a detector;
Fig. 5B is an enlarged view of the vicinity of the radiation source in Fig. 5A;
Fig. 5C is an enlarged diagram of pixels constituting a tomographic image of an inspection target in Fig. 5A;
Fig. 5D is a diagram illustrating paths of radiation emitted from the radiation source;
Fig. 6 illustrates an example of a linear attenuation coefficient table after interpolation;
Fig. 7 is a flowchart depicting an example of a tomographic image generation process executed by an information processing apparatus according to a first exemplary embodiment;
Fig. 8 is a diagram illustrating the setting of subset numbers and projection data numbers;
Fig. 9 is a flowchart depicting an example of a tomographic image generation process executed by an information processing apparatus according to a second exemplary embodiment;
Fig. 10A is a diagram illustrating a pencil beam method;
Fig. 10B is a diagram illustrating a cone beam method; and
Fig. 11 is a diagram illustrating a scattering angle.

DESCRIPTION OF EMBODIMENTS

Hereinafter, an exemplary embodiment of the present disclosure will be described in detail with reference to the drawings. It should be noted that the present exemplary embodiment is not intended to limit the present disclosure.

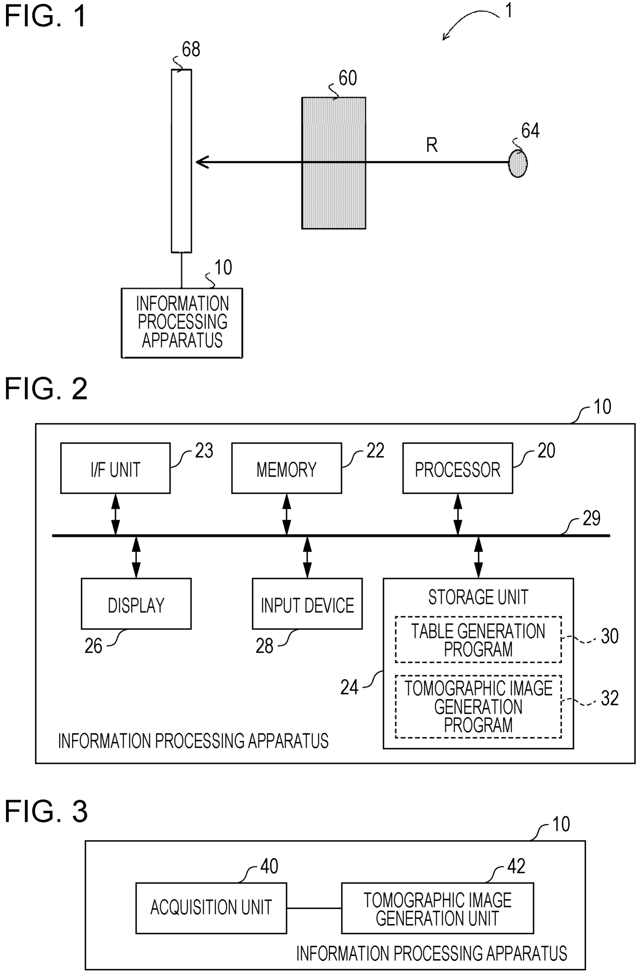

First, an example overall configuration of a tomographic image generation system 1 according to the present exemplary embodiment will be described. Fig. 1 is a block diagram illustrating an example overall configuration of the tomographic image generation system 1 according to the present exemplary embodiment. As illustrated in Fig. 1, the tomographic image generation system 1 according to the present exemplary embodiment includes an information processing apparatus 10, an inspection target 60, a radiation source 64, and a detector 68. While Fig. 1 illustrates one detector 68, the number of detectors 68 included in the tomographic image generation system 1 is not limited. For example, the detector 68 may be provided for each irradiation direction of radiation R. Alternatively, a plurality of detectors 68 may be arranged side by side such that one piece of projection data can be obtained from the plurality of detectors 68.

The information processing apparatus 10 according to the present exemplary embodiment generates a tomographic image of the inspection target 60 using projection data corresponding to each of a plurality of imaging positions at which the inspection target 60 is irradiated with the radiation R from different directions, one piece of projection data being obtained at each of at least two imaging positions among the plurality of imaging positions. The shape of the inspection target 60 is not specifically limited and may be any shape.

Specifically, the information processing apparatus 10 generates a tomographic image of the inspection target 60, based on attenuation information of the inspection target 60 and projection data obtained from the detector 68 when the inspection target 60 is irradiated with the radiation R from each of at least two directions.

The attenuation information refers to information on the linear attenuation coefficient of a member included in the inspection target 60. The linear attenuation coefficient is the probability of interaction of the radiation R per unit travel distance in the inspection target 60, and is the rate of decrease in the intensity or the number of photons of the radiation R. The linear attenuation coefficient changes depending on the energy of radiation. The radiation R emitted from the radiation source 64 has an energy distribution. Thus, the attenuation information preferably includes information on linear attenuation coefficients for at least two energy bands of the radiation R with which the inspection target 60 is irradiated.

The projection data is the total sum of the energies of a plurality of energy bands of the radiation R incident on each detector 68. The information processing apparatus 10 according to the present exemplary embodiment can solve problems such as a reduction in the image quality of a tomographic image and the occurrence of an artifact, by utilizing information on the linear attenuation coefficients of the inspection target 60 for the plurality of energy bands of the radiation R with which the inspection target 60 is irradiated.

When passing through the inspection target 60, the radiation R is attenuated due to interactions with the inspection target 60, such as photoelectric effect, coherent scattering (Thomson scattering), incoherent scattering (Compton scattering), and electron pair generation, and a portion of the attenuated radiation is generated as new radiation (scattered radiation).

The detector 68 detects the radiation R (hereinafter referred to as direct radiation) emitted from the radiation source 64 and passing through the inspection target 60, and also detects radiation newly generated (scattered) due to the interactions described above. That is, the projection data obtained from the detector 68 includes data based on the direct radiation as well as data based on the scattered radiation. In other words, the projection data obtained from the detector 68 is affected by the scattered radiation. Thus, the attenuation information preferably includes scattering information in order to reduce the effect of the scattered radiation included in the projection data. The probability of occurrence of scattering and the angle of scattering can be theoretically calculated by using, for example, the Klein-Nishina formula (Klein-Nishida model) for Compton scattering. However, the probability of occurrence of scattering and the angle of scattering vary depending on the type or the like of a member included in the inspection target 60. Thus, the attenuation information preferably includes scattering information of each member included in the inspection target 60.

The probability of occurrence of scattering and the angle of scattering also change depending on the energy of the radiation R to be emitted. Thus, the attenuation information preferably includes scattering information for at least two energy bands. In Compton scattering, the energy after scattering changes depending on the scattering angle, and the attenuation information preferably includes scattering information for at least two energy bands in order to enable calculation of multiple scattering (second-order scattering, third-order scattering, etc.) that occurs after the energy changes. By using the scattering information of the inspection target 60, the information processing apparatus 10 can generate a tomographic image with higher accuracy.

Hereinafter, the generation of a tomographic image of the inspection target 60 by the information processing apparatus 10 of the tomographic image generation system 1 according to the present exemplary embodiment will be described in detail for each exemplary embodiment.

First Exemplary Embodiment

Configuration of Information Processing Apparatus

First, the hardware configuration of the information processing apparatus 10 according to the present exemplary embodiment will be described. Fig. 2 is a block diagram illustrating an example hardware configuration of the information processing apparatus 10 according to the present exemplary embodiment. As illustrated in Fig. 2, the information processing apparatus 10 includes a processor 20 such as a central processing unit (CPU), a memory 22, an interface (I/F) unit 23, a storage unit 24, a display 26, and an input device 28. The processor 20, the memory 22, the I/F unit 23, the storage unit 24, the display 26, and the input device 28 are connected to each other via a bus 29, such as a system bus or a control bus, such that various kinds of information can be exchanged.

The processor 20 reads various programs stored in the storage unit 24, including a table generation program 30 and a tomographic image generation program 32, into the memory 22 and executes processes according to the read programs. Accordingly, the processor 20 performs control for generating a tomographic image. The memory 22 is a work memory for the processor 20 to execute a process.

The table generation program 30 and the tomographic image generation program 32 to be executed by the processor 20 are stored in the storage unit 24. Specific examples of the storage unit 24 include a hard disk drive (HDD) and a solid state drive (SSD).

The I/F unit 23 communicates various kinds of information with the detector 68 via wireless or wired communication. The display 26 and the input device 28 function as a user interface. The display 26 provides a user with various kinds of information related to sample analysis. The display 26 is not specifically limited, and examples thereof include a liquid crystal monitor and a light emitting diode (LED) monitor. The input device 28 is operated by the user to input various instructions for generating a tomographic image. The input device 28 is not specifically limited, and examples thereof include a keyboard, a touch pen, and a mouse. The information processing apparatus 10 employs a touch panel display that integrates the display 26 and the input device 28.

Fig. 3 is a functional block diagram illustrating an example configuration of the information processing apparatus 10. As illustrated in Fig. 3, the information processing apparatus 10 includes an acquisition unit 40 and a tomographic image generation unit 42. When the processor 20 executes the table generation program 30 or the tomographic image generation program 32, the processor 20 functions as the acquisition unit 40 and the tomographic image generation unit 42.

The acquisition unit 40 has a function of acquiring a plurality of pieces of projection data obtained from the detector 68 when the inspection target 60 is irradiated with the radiation R in different directions from the radiation source 64. As an example, in the information processing apparatus 10 according to the present exemplary embodiment, after imaging of the inspection target 60 is performed, a plurality of pieces of projection data with different irradiation directions of the radiation R are acquired from the detector 68 at any timing and stored in the storage unit 24. In addition, when performing a tomographic image generation process, the acquisition unit 40 acquires the plurality of pieces of projection data with different irradiation directions of the radiation R, which are stored in the storage unit 24, and outputs the plurality of pieces of projection data to the tomographic image generation unit 42.

The tomographic image generation unit 42 has a function of generating a tomographic image of the inspection target 60, based on a plurality of pieces of projection data with different irradiation directions of the radiation R, which are acquired by the acquisition unit 40, and attenuation information of the inspection target 60. The tomographic image generation unit 42 according to the present exemplary embodiment has a function of generating various tables (described in detail below) necessary to generate a tomographic image.

Next, the operation of the information processing apparatus 10 according to the present exemplary embodiment will be described in detail.

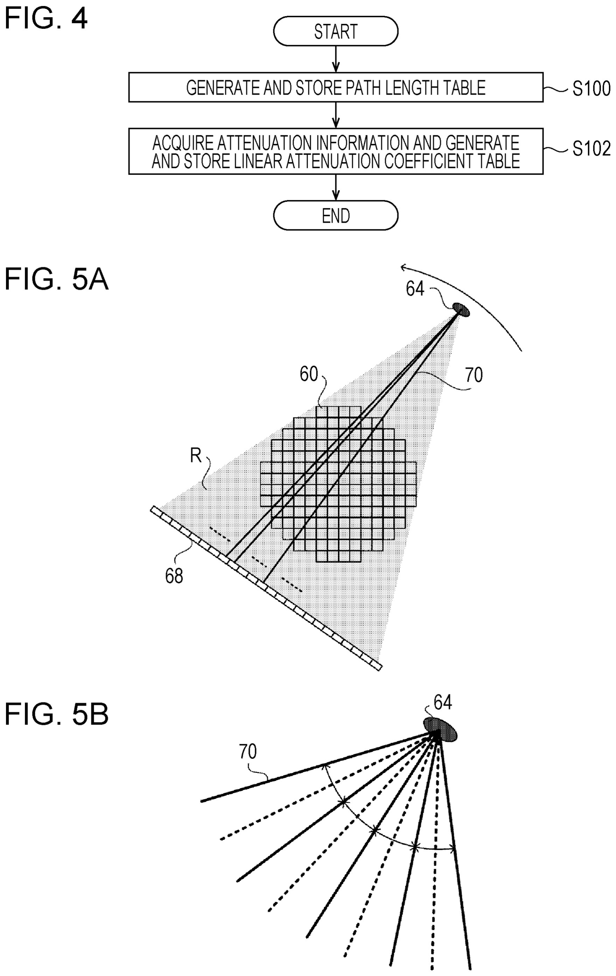

As described above, the information processing apparatus 10 according to the present exemplary embodiment generates various tables necessary to generate a tomographic image before generating the tomographic image. Fig. 4 is a flowchart illustrating an example of a table generation process executed by the information processing apparatus 10 according to the present exemplary embodiment. The information processing apparatus 10 executes the table generation program 30 stored in the storage unit 24 to execute the table generation process illustrated in Fig. 4.

In step S100 illustrated in Fig. 4, the tomographic image generation unit 42 generates a path length table of the radiation R, based on the geometric arrangement of the radiation source 64 and the detector 68, and stores the generated table.

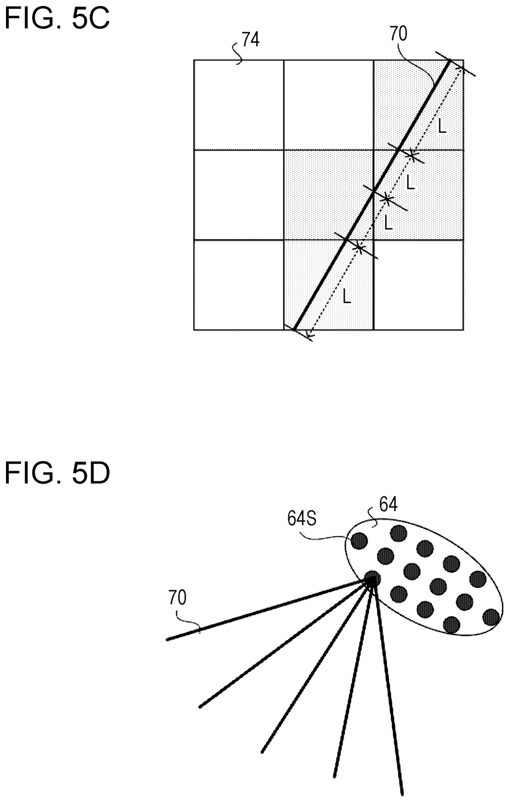

In the present exemplary embodiment, a fan beam method will be described as an example of the scan method. As illustrated in Fig. 5A, paths 70 of the radiation R from the radiation source 64 to the detector 68 are set. Fig. 5B is an enlarged view of the vicinity of the radiation source 64. As illustrated in Fig. 5B, the paths 70 are set at equal intervals with a minute angle. As more paths 70 are set, or in other words, as the angle is set to be smaller, a tomographic image can be generated with higher accuracy, but more calculation time is required. For each detector 68, paths 70 incident on the detector 68 are extracted, and the numbers of paths 70 are tabulated. Then, for each path 70, as illustrated in Fig. 5C, pixels 74 through which the path 70 passes are extracted, the number of extracted pixels 74 and the numbers of the pixels 74 are tabulated, and path lengths L for the respective pixels 74 are determined and tabulated. Fig. 5C is an enlarged diagram of pixels constituting a tomographic image of the inspection target 60 in Fig. 5A, in which pixels through which the path 70 passes are indicated by shading, and the path length in each pixel is indicated. In the example illustrated in Fig. 5C, the path 70 passes through four pixels. A pixel number is a number for identifying each pixel of a tomographic image and is originally given in two dimensions of x and y, but is represented as a one-dimensional number because an increase in the number of dimensions makes it complicated to refer to a table.

The tomographic image generation unit 42 generates, for example, the following tables.

· Table num_path(no_s, no_d): a table of the number of paths 70 incident on the detector no_d in the scan no_s. Note that "no_s" represents the identification number of a scan, and a scan with the identification number no_s is referred to as a "scan no_s". In the case of a fan beam, "no_s" represents an imaging identification number for each direction during imaging with the rotation of the radiation source 64 and the detector 68. "no_d" represents the identification number of a detector 68, and a detector 68 with the identification number no_d is referred to as a "detector no_d".
· Table num_pix(no_s, no_d, no_path): a table of the number of pixels through which the path no_path incident on the detector no_d in the scan no_s passes. "no_path" represents the identification number of a path 70, and a path 70 with the identification number no_path is represented as a "path no_path".
· Table no_pix(no_s, no_d, no_path, no_pix_path): a table of the identification number of the (no_pix_path)-th pixel through which the path no_path incident on the detector no_d in the scan no_s passes. "no_pix_path" represents the identification number of a pixel along the path no_path incident on the detector no_d in the scan no_s, and numbers are assigned in ascending order, starting from the one closest to the radiation source 64.

· Table plen_pix(no_s, no_d, no_path, no_pix_path): a table of path lengths for the (no_pix_path)-th pixel through which the path no_path incident on the detector no_d in the scan no_s passes.

Further, for each subset (described in detail below) obtained by dividing all scans into several sets (subsets), the total sum of path lengths through each pixel is determined for all the paths 70 incident on all the detectors 68 in all the scans belonging to the subset, and is tabulated.

For example, the following table is generated.

• Table plen_pix_sub(no_sub, no_pix): a table of the total sum of path lengths through the pixel no_pix for all the paths 70 incident on all the detectors 68 in all the scans belonging to a subset no_sub. "no_sub" represents the identification number of a subset, and a subset with the identification number no_sub is referred to as a "subset no_sub".

In practice, as illustrated in Fig. 5D, the radiation source 64 is not a point, but has a spatial extent. In other words, to ensure the dose of the radiation R, that is, to ensure the brightness or the density difference of the projection data, the radiation source 64 has a certain size. Due to the extent of the radiation source 64, blurring occurs in the projection data and affects the spatial resolution. To generate an accurate tomographic image from projection data including such blurring, it is preferable to set the path 70 of the radiation R on the premise of the spatial extent of the radiation source 64. For example, as illustrated in Fig. 5D, a plurality of (in Fig. 5D, 14) radiation sources 64S may be set at predetermined intervals in a spatially extending predetermined region (the radiation source 64), and paths 70 of the radiation R may be set from each of the radiation sources 64S to the detectors 68. Also in a case where a plurality of radiation sources 64S are set, no need exists to generate a table while distinguishing the radiation sources 64S. It is sufficient to generate the table described above by collecting the paths 70 from all the radiation sources 64S.

Next, in step S102, the tomographic image generation unit 42 interpolates the linear attenuation coefficients of the members included in the inspection target 60 to generate a linear attenuation coefficient table, and stores the generated table.

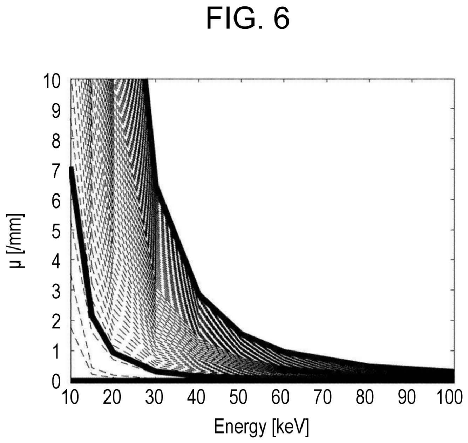

Fig. 6 illustrates an example of the linear attenuation coefficient table f(no_f, no_e) after the interpolation. "no_f" represents the identification number of a linear attenuation coefficient, and "no_e" represents the identification number of energy corresponding to a wavelength of the radiation R, and f(no_f, no_e) represents a table of linear attenuation coefficients with the respective linear attenuation coefficient numbers no_f at the energy no_e. The tomographic image generation unit 42 interpolates the linear attenuation coefficients of the members included in the inspection target 60 at each energy to generate f(no_f, no_e). In Fig. 6, three thick lines are provided, which indicate the linear attenuation coefficients of air, aluminum, and iron in order from the smallest to the largest. The values of these linear attenuation coefficients are derived from "Tables of X-Ray Mass Attenuation Coefficients and Mass Energy-Absorption Coefficients from 1 keV to 20 MeV for Elements Z = 1 to 92 and 48 Additional Substances of Dosimetric Interest, NIST, J.H. Hubbell and S.M. Seltzer." The surroundings of the inspection target 60 are filled with a substance corresponding to the environment in which the inspection target 60 is present, and a tomographic image of the inspection target 60 includes pixels corresponding to the members included in the inspection target 60 and also includes pixels corresponding to the substance with which the surroundings of the inspection target 60 are filled. In the present exemplary embodiment, accordingly, the entire region of the tomographic image is considered to be the inspection target 60, and the substance with which the surroundings of the original inspection target 60 are filled is also considered to be a member included in the inspection target 60. In the example illustrated in Fig. 6, the members included in the original inspection target 60 are aluminum and iron. However, since the surroundings of the original inspection target 60 are filled with air, the air is also considered to be a member included in the inspection target 60 (in the tomographic image of the inspection target 60, each pixel corresponds to any one of aluminum and iron included in the inspection target 60 or the air with which the surroundings of the inspection target 60 are filled).

In Fig. 6, each thin wavy line indicates a virtual linear attenuation coefficient generated by interpolation from the linear attenuation coefficients of air, aluminum, and iron. The virtual linear attenuation coefficient is determined by linearly interpolating the linear attenuation coefficients of air, aluminum, and iron at each energy. In the example in Fig. 6, the linear attenuation coefficients of aluminum and iron are equally divided by 80 at each energy to determine virtual linear attenuation coefficients between aluminum and iron. This method provides equal differences between the total sum values or mean values of adjacent virtual linear attenuation coefficients between aluminum and iron at all energies. The maximum value of the linear attenuation coefficient numbers no_f is represented as num_f. In the example in Fig. 6, num_f= 85, and the linear attenuation coefficient thereof is the linear attenuation coefficient of iron. Further, the differences between the total sum values or mean values of adjacent virtual linear attenuation coefficients between air and aluminum at all energies are set to be equal to the differences between the total sum values or mean values of adjacent virtual linear attenuation coefficients between aluminum and iron at all energies. The interval between virtual linear attenuation coefficients of air and aluminum and the interval between virtual linear attenuation coefficients of aluminum and iron may be any interval. A smaller interval allows a tomographic image to be generated with higher accuracy, but requires more calculation time. As illustrated in Fig. 6, furthermore, the differences between the total sum values or mean values of adjacent virtual linear attenuation coefficients between air and aluminum and between aluminum and iron at all energies may be made equal, or the differences between the total sum values of energy $\times$ linear attenuation coefficients at all energies may be made equal in consideration of energy. For air, the value of the linear attenuation coefficient may be approximately set to 0.0 at all energies. In other words, the member no_f whose value of the linear attenuation coefficient is 0.0 at all energies may be considered to be air.

The present exemplary embodiment is based on the premise that the magnitude relationship between the linear attenuation coefficients of the members included in the inspection target 60 is the same at all energies, that is, the magnitude relationship is not reversed, and the virtual linear attenuation coefficient increases as the linear attenuation coefficient number no_f increases at any energy.

When the processing of step S102 is completed, the table generation process illustrated in Fig. 4 ends.

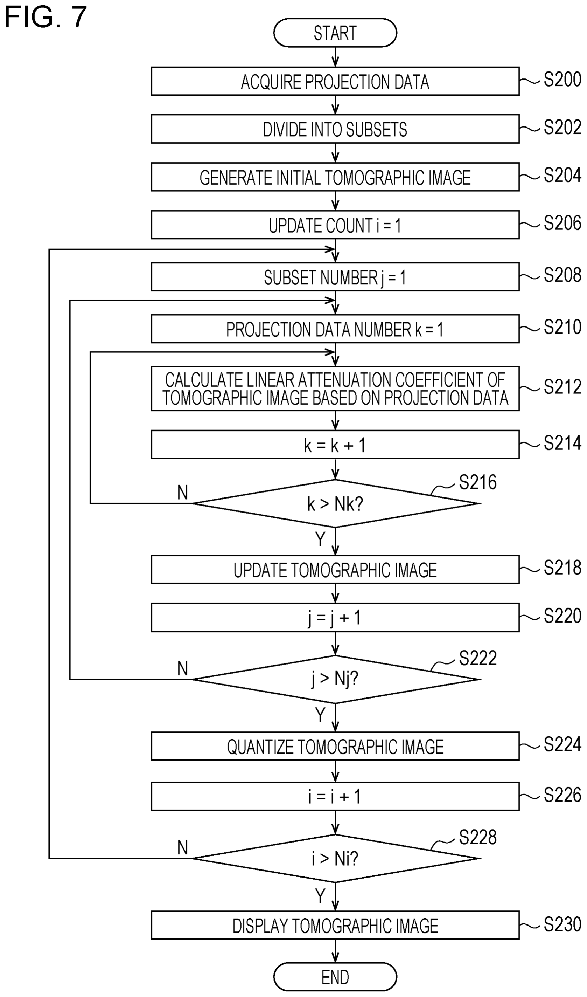

When the table generation process illustrated in Fig. 4 is completed in this way, the information processing apparatus 10 generates a tomographic image. Fig. 7 is a flowchart illustrating an example of a tomographic image generation process executed by the information processing apparatus 10 according to the present exemplary embodiment. The information processing apparatus 10 executes the tomographic image generation program 32 stored in the storage unit 24 to perform the tomographic image generation process illustrated in Fig. 7.

In step S200 illustrated in Fig. 7, as described above, the acquisition unit 40 acquires a plurality of pieces of projection data from the storage unit 24 and outputs the pieces of projection data to the tomographic image generation unit 42.

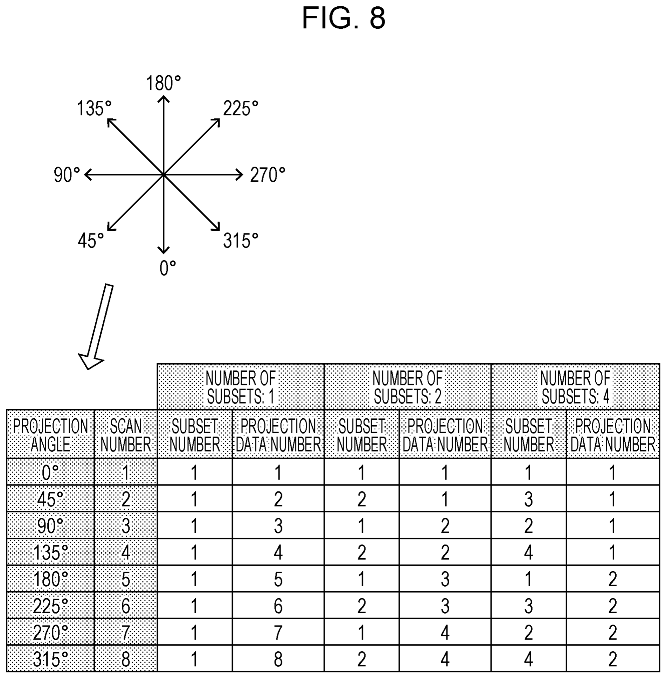

Next, in step S202, the tomographic image generation unit 42 divides the pieces of projection data in all directions (all scans) into several sets (subsets). For example, when the number of pieces of projection data (the number of scans or the number of projection directions) is eight, subset numbers and projection data numbers can be set as in an example illustrated in Fig. 8. As in the example illustrated in Fig. 8, preferably, pieces of projection data with projection directions as close to opposite directions as possible are included in the same subset. Preferably, pieces of projection data with projection directions as close to orthogonal as possible are included in the same subset. It is also preferable to set subset numbers in the order of subsets with projection directions as close to orthogonal as possible (to update the tomographic image (described in detail below)).

In the tomographic image generation process illustrated in Fig. 7, the tomographic image generation unit 42 updates a tomographic image using only pieces of projection data belonging to a subset, and repeats the update for each subset to perform the update for all the subsets. After that, at the point in time when the linear attenuation coefficients of the tomographic image are quantized to the values of the members included in the inspection target 60, the tomographic image generation unit 42 increments the total update count by one. The tomographic image generation unit 42 completes the generation of a tomographic image at the point in time when the entire update is performed a predetermined number of times.

In step S204, the tomographic image generation unit 42 generates an initial tomographic image (described in detail below). Next, in step S206, the tomographic image generation unit 42 sets a total update count i to "1".

Next, in step S208, the tomographic image generation unit 42 initializes a subset number j to "1". The subset number j is initialized for each update count i. Next, in step S210, the tomographic image generation unit 42 initializes a projection data number k to "1". The projection data number k is initialized for each subset j.

Next, in step S212, the tomographic image generation unit 42 calculates a linear attenuation coefficient of each pixel of a tomographic image, based on the projection data with a scan number corresponding to a combination of the subset number j and the projection data number k (described in detail below).

Next, in step S214, the tomographic image generation unit 42 increments the projection data number k (k = k + 1). Next, in step S216, the tomographic image generation unit 42 determines whether the projection data number k exceeds the total number Nk of pieces of projection data belonging to the subset j (k > Nk). The determination of step S216 remains negative until the projection data number k exceeds the total number Nk. Then, the process returns to step S212, and the calculation of the linear attenuation coefficients of the tomographic image is repeatedly performed. On the other hand, if the projection data number k exceeds the total number Nk, the determination of step S216 is positive, and then the process proceeds to step S218.

In step S218, the tomographic image generation unit 42 updates the tomographic image based on the linear attenuation coefficient calculated in step S212 described above.

Next, in step S220, the tomographic image generation unit 42 increments the subset number j (j = j + 1). Next, in step S222, the tomographic image generation unit 42 determines whether the subset number j exceeds the total number Nj of subsets (j > Nj). The determination of step S222 remains negative until the subset number j exceeds the total number Nj. Then, the process returns to step S210, and the update of the tomographic image is repeatedly performed. On the other hand, if the subset number j exceeds the total number Nj, the determination of step S222 is positive, and then the process proceeds to step S224.

In step S224, the tomographic image generation unit 42 quantizes the tomographic image updated in step S218 described above (described in detail below).

Next, in step S226, the tomographic image generation unit 42 increments the update count i (i = i + 1). Next, in step S228, the tomographic image generation unit 42 determines whether the update count i exceeds a predetermined number Ni (i > Ni). The determination of step S228 remains negative until the update count i exceeds the predetermined number Ni. Then, the process returns to step S208, and the update and quantization of the tomographic image are repeatedly performed. On the other hand, if the update count i exceeds the predetermined number Ni, the determination of step S228 is positive, and then the process proceeds to step S230.

Next, in step S230, the tomographic image generation unit 42 displays the tomographic image generated by the processing described above on the display 26. When the processing of step S230 is completed, the tomographic image generation process illustrated in Fig. 7 ends.

The details of steps S204, S212, S218, and S224 will be described hereinafter.

The details of step S204 will be described. In step S204, as described above, an initial tomographic image no_f(no_pix) is generated. no_f(no_pix) represents the linear attenuation coefficient number no_f of the pixel no_pix, and no_pix takes any value from 1 to num_pix_t, where num_pix_t denotes the total number of pixels in the tomographic image. That is, the tomographic image is an image having pixels assigned linear attenuation coefficient numbers, and linear attenuation coefficients at respective energies corresponding to the respective linear attenuation coefficient numbers are given by the linear attenuation coefficient table f(no_f, no_e) generated in advance. The mean value is set as the initial value of no_f(no_pix). For example, in the example in Fig. 6, the linear attenuation coefficients of air, aluminum, and iron are interpolated to set the virtual linear attenuation coefficients with linear attenuation coefficient numbers 1 to 85, and the mean value, namely, 43, is set.

The details of step S212 will be described. The tomographic image generation unit 42 calculates a linear attenuation coefficient of the tomographic image, based on pieces of projection data g(no_s, 1) to g(no_s, num_d). The scan number no_s is determined so as to correspond to the combination of the subset number j and the projection data number k (see Fig. 8). "num_d" represents the number of detectors 68.

A calculation method based on the projection data g(no_s, no_d) of the detector no_d will be described. First, virtually simulated projection data g_v (hereinafter referred to as virtual projection data g_v) of the detector no_d in the scan no_s is determined using Formula (1) below, based on the current tomographic image.

$$g\_v = I(1) + I(2) + ... + I(\text{num\_path}(no\_s, no\_d)) ... (1)$$

In Formula (1) above, num_path(no_s, no_d) is the number of paths 70 incident on the detector no_d in the scan no_s, which is determined in advance.

In Formula (1) above, I(no_path) represents the intensity of radiation along the path no_path incident on a detector and is determined using Formula (2) below.

$$I(\text{no\_path}) = (e(1) \times I\_e(1)) + (e(2) \times I\_e(2)) + ... + (e(\text{num\_e}) \times I\_e(\text{num\_e})) ... (2)$$

In Formula (2) above, e(1), e(2), ..., and e(num_e) indicate energies and are known. I(no_path) can be considered to be the total energy of photons having num_e kinds of energies along the path no_path incident on the detector 68.

The intensity I_e(no_e) of the radiation R at the energy e(no_e) in Formula (2) above is determined using Formula (3) below.

I_e(no_e) = I0_e(no_e) × exp(-{(f(no_f_ext(1), no_e) × plen_pix(no_s, no_d, no_path, 1)) + (f(no_f ext(2), no_e) × plen_pix(no_s, no_d, no_path, 2)) + ... + (f(no_f_ext(num_pix(no_s, no_d, no_path)), no_e) × plen_pix(no_s, no_d, no_path, num_pix(no_s, no_d, no_path)))}), where

$$no\_f\_ext(\text{no\_pix\_path}) = no\_f(no\_pix(no\_s, no\_d, no\_path, no\_pix\_path)) ... (3)$$

Formula (3) above represents the intensity I_e(no_e) of the radiation R with the intensity I0_e(no_e) at the energy e(no_e) emitted from the radiation source 64, passing through each pixel while being attenuated, and incident on the detector 68.

In Formula (3) above,
f(no_f(no_pix(no_s, no_d, no_path, no_pix_path)), no_e) of f(no_f_ext(no_pix_path), no_e) × plen_pix(no_s, no_d, no_path, no_pix_path) = f(no_f(no_pix(no_s, no_d, no_path, no_pix_path)), no_e) × plen_pix(no_s, no_d, no_path, no_pix_path) represents the linear attenuation coefficient f at the energy no_e corresponding to the linear attenuation coefficient number no_f of the (no_pix_path)-th pixel no_pix through which the path no_path incident on the detector no_d in the scan no_s passes. Further, plen_pix(no_s, no_d, no_path, no_pix_path) represents the path length plen_pix in the (no_pix_path)-th pixel no_pix through which the path no_path incident on the detector no_d in the scan no_s passes.

The intensity I0_e(no_e) in Formula (3) above represents the total sum of the numbers of photons (at the energy e(no_e)) emitted per unit time over the minute angular ranges in Fig. 5B, that is, angular ranges surrounded by broken lines and centered on the paths 70. That is, as the interval of the angles of the paths in Fig. 5B decreases, or in other words, as more paths 70 are set, I0_e(no_e) is set to a smaller value. In Fig. 5B, to facilitate understanding of the description, only minute angular ranges within a plane defined by the arrangement directions of the radiation source 64 and the detector 68 (within a two-dimensional fan beam plane) are depicted by broken lines. In practice, the radiation R is three-dimensionally emitted from the radiation source 64, and each detector detects radiation incident on a two-dimensional plane of the detector. That is, to be accurate, the intensity I0_e(no_e) in Formula (3) above represents the total sum of the numbers of photons emitted per unit time over the minute angular ranges in Fig. 5B (the angular ranges surrounded by the broken lines) and a minute angular range of the detector in a direction perpendicular to the minute angular ranges (in a direction perpendicular to the fan beam plane). I0_e(0), I0_e(1), ..., and I0_e(num_e) are determined by the radiation source 64 and are known.

The virtual projection data g_v determined using Formula (1) above is compared with the actually measured projection data g(no_s, no_d). If the virtual projection data g_v is larger, the linear attenuation coefficient numbers of all the pixels through which all the paths 70 incident on the detector no_d in the scan no_s pass are increased by 1 to determine the virtual projection data g_v again, and the determined virtual projection data g_v is compared with the actually measured projection data g(no_s, no_d). The process of increasing the linear attenuation coefficient number by 1, recalculating the virtual projection data g_v, and comparing the virtual projection data g_v with the projection data g(no_s, no_d) in the way described above is repeatedly performed until the virtual projection data g_v becomes less than or equal to the actually measured projection data g(no_s, no_d). Then, among the virtual projection data g_v immediately before becoming less than or equal to the actually measured projection data g(no_s, no_d) and the virtual projection data g_v that becomes less than or equal to the actually measured projection data g(no_s, no_d), the virtual projection data g_v closer to the actually measured projection data g(no_s, no_d) is used. The increment of the linear attenuation coefficient number used to calculate the virtual projection data g_v is stored as df(no_s, no_d). To increase the linear attenuation coefficient number by α to recalculate the virtual projection data g_v, in Formula (3) above, no_f_ext(no_pix_path) is set to no_f(no_pix(no_s, no_d, no_path, no_pix_path)) + α instead of no_f(no_pix(no_s, no_d, no_path, no_pix_path)) to recalculate I_e(no_e). Then, the intensity I(no_path) is recalculated from recalculated I_e(1), I_e(2), ..., and I_e(num_e) using Formula (2) above, and the virtual projection data g_v is recalculated from recalculated I(1), I(2), ..., and I(num_path(no_s, no_d)) using Formula (1) above.

If the pixels no_pix(no_s, no_d, no_path, 1) to no_pix(no_s, no_d, no_path, num_pix(no_s, no_d, no_path)) include a pixel for which no_f(no_pix(no_s, no_d, no_path, no_pix_path)) + α exceeds the maximum value of the linear attenuation coefficient numbers, the linear attenuation coefficient number of that pixel is fixed to the maximum value. For example, in the example in Fig. 6, the maximum value of the linear attenuation coefficient numbers is 85. Thus, for a pixel whose linear attenuation coefficient number exceeds 85 as a result of increasing the linear attenuation coefficient number by α, the linear attenuation coefficient number is fixed to 85. When the virtual projection data g_v is smaller than the actual projection data g(no_s, no_d), conversely, the linear attenuation coefficient number of all the pixels through which all the paths 70 incident on the detector no_d in the scan no_s pass is decreased by 1 to recalculate the virtual projection data g_v. This process is repeatedly performed until the virtual projection data g_v becomes greater than or equal to the actually measured projection data g(no_s, no_d). Then, among the virtual projection data g_v immediately before becoming greater than or equal to the actually measured projection data g(no_s, no_d) and the virtual projection data g_v that becomes greater than or equal to the actually measured projection data g(no_s, no_d), the virtual projection data g_v closer to the actually measured projection data g(no_s, no_d) is used, and the amount of decrease in the linear attenuation coefficient number during the calculation is stored as df(no_s, no_d). At this time, df(no_s, no_d) is a negative value. Also when the linear attenuation coefficient number is decreased, for a pixel whose linear attenuation coefficient number is smaller than the minimum value of 1 as a result of the decrease, the linear attenuation coefficient number is fixed to 1.

Through the calculation described above, the increments df(no_s, 1) to df(no_s, num_d) of the linear attenuation coefficient number can be determined based on the pieces of projection data g(no_s, 1) to g(no_s, num_d).

The details of step S218 will be described. After the calculation of the differences df(no_s, 1) to df(no_s, num_d) in linear attenuation coefficient number is completed for all the pieces of projection data belonging to the subset j, the tomographic image generation unit 42 updates the tomographic image based on the differences.

The focus is placed on a certain pixel no_pix, and it is assumed that a path 70 incident on the detector no_d in the scan no_s passes through this pixel. At this time, the difference df(no_s, no_d) is an estimated value of a difference in linear attenuation coefficient number for the path 70 incident on the detector no_d in the scan no_s and passing through the pixel no_pix. Accordingly, it is necessary to determine the mean value by weighting the estimated values of the differences in linear attenuation coefficient number for all the paths 70 passing through the pixel no_pix by respective path lengths. First, df_ave(no_pix) is initialized to have a value of "0" for all the pixels, where df_ave(no_pix) denotes a tomographic image having the mean value of the differences. Here, df_ave(no_pix) represents the mean value of the differences in linear attenuation coefficient number for the pixel no_pix, and no_pix is any value from 1 to the total number of pixels, namely, num_pix_t. Then, for all the pieces of projection data belonging to the subset j, the differences df(no_s, 1) to df(no_s, num_d) in linear attenuation coefficient number are added to the respective pixels of df_ave while being weighted by the path lengths. Finally, the value of each pixel of df_ave is divided by the total sum plen_pix_sub of the path lengths of the respective pixels for which the table has been generated in advance to determine the mean value of the differences for the respective pixels, and the mean value is added to the tomographic image before the update to update the tomographic image.

The content of the addition process will be described in detail using, as an example, the pieces of projection data in the scan no_s belonging to the subset j. The number of pixels through which the path no_path incident on the detector no_d in the scan no_s passes is num_pix(no_s, no_d, no_path). In the tomographic image df_ave with the mean value of the differences, the value given by difference × path length, that is, df(no_s, no_d) × plen_pix(no_s, no_d, no_path, no_pix_path), is added to the value of the (no_pix_path)-th pixel no_pix(no_s, no_d, no_path, no_pix_path) through which the path no_path incident on the detector no_d in the scan no_s passes. In a similar way, in the tomographic image df_ave with the mean value of the differences, the values given by difference df(no_s, no_d) × path lengths (plen_pix(no_s, no_d, no_path, 1) to plen_pix(no_s, no_d, no_path, num_pix(no_s, no_d, no_path))) are each added to the values of all the num_pix(no_s, no_d, no_path) pixels through which the path no_path passes. If the pixels no_pix(no_s, no_d, no_path, 1) to no_pix(no_s, no_d, no_path, num_pix(no_s, no_d, no_path)) include a pixel whose linear attenuation coefficient number exceeds the maximum value (in the example in Fig. 6, a pixel whose linear attenuation coefficient number exceeds the maximum value of 85) as a result of adding the difference df(no_s, no_d) to the linear attenuation coefficient numbers before the update, the difference is corrected for that pixel so that the result of the addition becomes the maximum value, and then the corrected difference is added to df_ave. Similarly, when a pixel whose linear attenuation coefficient number becomes smaller than the minimum value of 1 as a result of adding the difference df(no_s, no_d) to the linear attenuation coefficient numbers before the update is included, the difference is corrected for that pixel so that the result of the addition becomes 1, and then the corrected difference is added to df_ave. The addition process described above is performed for all the paths 1 to num_path(no_s, no_d) incident on the detector no_d in the scan no_s. Then, the addition process described above is performed for all the detectors 1 to num_d in the scan no_s. The addition process for the pieces of projection data in the scan no_s belonging to the subset j has been described above. This addition process is performed for all the pieces of projection data belonging to the subset j. Finally, df_ave(1) to df_ave(num_pix_t) generated through the addition process described above are divided by the total sums plen_pix_sub(j, 1) to plen_pix_sub(j, num_pix_t) of the path lengths of the respective pixels to determine tomographic images df_ave(1) to df_ave(num_pix_t) with the mean values of the differences in linear attenuation coefficient number, and the tomographic images df_ave(1) to df_ave(num_pix_t) are added to the tomographic images no_f(1) to no_f(num_pix_t) before the update to update the tomographic images. The tomographic image df_ave with the mean value of the differences is added to the tomographic image no_f, and then the result is rounded to the nearest integer value.

The details of step S224 will be described. After updating the tomographic images for all the subsets, the tomographic image generation unit 42 quantizes the linear attenuation coefficients of the tomographic images to the values of the members included in the inspection target 60. Specifically, the linear attenuation coefficient number no_f(no_pix) of each pixel no_pix in the tomographic image is corrected to the linear attenuation coefficient number of the member included in the inspection target 60 that is closest to the linear attenuation coefficient number no_f(no_pix). For example, in the example in Fig. 6, the members included in the inspection target 60 are air, aluminum, and iron, and the linear attenuation coefficient number of air is 1 (minimum value), the linear attenuation coefficient number of aluminum is 5, and the linear attenuation coefficient number of iron is 85 (maximum value). In this case, in the tomographic image no_f, the linear attenuation coefficient numbers of the respective pixels are corrected to the linear attenuation coefficient number of air for pixels having linear attenuation coefficient numbers less than or equal to 3 or less than 3, to the linear attenuation coefficient number of aluminum for pixels having linear attenuation coefficient numbers greater than or equal to 4, greater than or equal to 3 and less than or equal to 45, or less than 45, or to the linear attenuation coefficient number of iron for pixels having linear attenuation coefficient numbers greater than or equal to 46 or greater than or equal to 45.

As described above, according to the present exemplary embodiment, a high-accuracy tomographic image of the inspection target 60 can be generated regardless of the number of pieces of projection data.

Second Exemplary Embodiment

The present exemplary embodiment describes a tomographic image generation process different from that in the first exemplary embodiment. Also in the present exemplary embodiment, as in the first exemplary embodiment, the table generation process (see Fig. 4) is executed to generate various tables before a tomographic image is generated. Specifically, the tomographic image generation unit 42 generates the table num_path(no_s, no_d) for the number of paths 70, the table num_pix(no_s, no_d, no_path) for the number of pixels, the table no_pix(no_s, no_d, no_path, no_pix_path) for the numbers of the pixels, and the table plen_pix(no_s, no_d, no_path, no_pix_path) for the path lengths. The tomographic image generation unit 42 also generates a linear attenuation coefficient table f(no_f, no_e). In the present exemplary embodiment, the table plen_pix_sub(no_sub, no_pix) for the total sum of path lengths belonging to the subset no_sub is not generated.

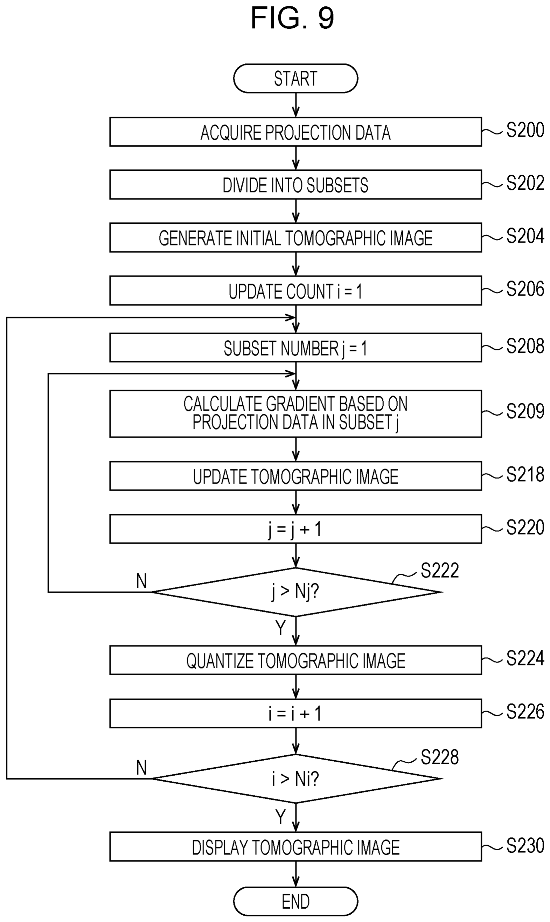

Fig. 9 is a flowchart illustrating an example of a tomographic image generation process executed by the information processing apparatus 10 according to the present exemplary embodiment. As illustrated in Fig. 9, the tomographic image generation process according to the present exemplary embodiment is different from the tomographic image generation process according to the first exemplary embodiment (see Fig. 7) in that the processing of step S209 is included instead of steps S210 to S216. The specific content of the processing of S218 after step S209 differs from that in the first exemplary embodiment.

In the present exemplary embodiment, an evaluation function is defined to evaluate the difference between the virtual projection data g_v determined from the generated tomographic image no_f and the actually measured projection data g(no_s, no_d). Then, the tomographic image is updated so that the evaluation function is decreased. Specifically, for each of the pixels 1 to num_pix_t in the tomographic image no_f, the change in evaluation function in response to a slight change in the value (linear attenuation coefficient number) of the tomographic image is determined, and this value is set as a gradient. Then, the tomographic image is updated based on the gradient determined for each of the pixels 1 to num_pix_t. The gradient is determined for each subset j, and the tomographic image is updated. Steps S209 and S218 will be described in detail hereinafter.

In step S209, the tomographic image generation unit 42 calculates the gradient based on the pieces of projection data belonging to the subset j. First, an evaluation function ef is defined in advance as follows.

$$ef = \Sigma\Sigma(g\_v(no\_s, no\_d) - g(no\_s, no\_d))^2,$$

where

the first $\Sigma$ is the total sum for the scan no_s belonging to the subset j,
the second $\Sigma$ is the total sum (total sum of 1 to num_d) for the detector no_d,
g_v(no_s, no_d) represents virtual projection data for the detector no_d in the scan no_s, and is determined from the tomographic image, and g(no_s, no_d) represents the actually measured projection data for the detector no_d in the scan no_s.          (4)

First, the respective pieces of virtual projection data g_v(no_s, no_d) are determined from the current tomographic image no_f using Formulas (1), (2), and (3) above, and then the evaluation function ef is determined using Formula (4) above. Next, no_f(1), that is, the value (linear attenuation coefficient number) of the pixel 1 in the tomographic image no_f, is hypothetically increased by a predetermined amount $\alpha$ or 1, the respective pieces of virtual projection data g_v(no_s, no_d) are determined again using Formulas (1), (2), and (3) above, and the evaluation function ef is determined again using Formula (4) above. If no_f(1) is increased by $\alpha$, when the pixel no_pix(no_s, no_d, no_path, no_pix_path) is equal to 1 in Formula (3) above, no_f_ext(no_pix_path) is set to no_f(no_pix(no_s, no_d, no_path, no_pix_path))+$\alpha$ instead of no_f(no_pix(no_s, no_d, no_path, no_pix_path)) to calculate the intensity I_e(no_e). In this way, the evaluation function ef before the increase in the value of the pixel 1 in the tomographic image no_f by $\alpha$ is subtracted from the evaluation function ef after the increase to determine a gradient $\Delta ef(1)$ for the pixel 1.

In the calculation of the gradient, it is desirable that the gradient be correctly computable even when no_f(1) + $\alpha$ exceeds the maximum value num_f of the linear attenuation coefficient numbers. Accordingly, the linear attenuation coefficient table f(no_f, no_e) used to calculate the gradient has additional $\alpha$ tables beyond the maximum value num_f of the linear attenuation coefficient numbers. That is, a number of tables equal to linear attenuation coefficient numbers 1 to num_f + $\alpha$ are held. At each energy no_e, an interval obtained by subtracting the linear attenuation coefficient f with the linear attenuation coefficient number num_f - 1 from the linear attenuation coefficient f with the linear attenuation coefficient number num_f is represented by $\Delta f$. Values obtained by adding $1 \times \Delta f$, $2 \times \Delta f$, ..., and $\alpha \times \Delta f$ to the linear attenuation coefficient f with the linear attenuation coefficient number num_f are assigned to values of the $\alpha$ additional tables.

In a similar way, the gradients $\Delta$ef(2), $\Delta$ef(3), ..., and $\Delta$ef(num_pix_t) for the pixels 2, 3, ..., and num_pix_t are determined. To quickly determine the evaluation function ef after the increase by $\alpha$, if none of the pixels no_pix(no_s, no_d, no_path, 1) to no_pix(no_s, no_d, no_path, num_pix(no_s, no_d, no_path)) through which the path no_path incident on the detector no_d in the scan no_s passes matches the pixel obtained as a result of the increase by $\alpha$ during the calculation of Formula (3) above, the calculation of Formulas (2) and (3) above may be omitted for that path 70, and the intensity I(no_path) of the radiation R along the path no_path may be set to the same value as the intensity I(no_path) before the increase by $\alpha$.

The evaluation function ef may be any function that can evaluate the difference between the projection data g_v determined from the tomographic image no_f and the actually measured projection data g(no_s, no_d). For example, the following function may be used.

$$ef = \Sigma\Sigma \ abs(g\_v(no\_s, no\_d) - g(no\_s, no\_d)),$$

where

the first $\Sigma$ is the total sum for the scan no_s belonging to the subset j,
the second $\Sigma$ is the total sum (total sum of 1 to num_d) for the detector no_d,
abs(x) represents the absolute value of x,
g_v(no_s, no_d) represents virtual projection data for the detector no_d in the scan no_s, and is determined from the tomographic image, and g(no_s, no_d) represents the actually measured projection data for the detector no_d in the scan no_s.          (5)

The processing of step S218 according to the present exemplary embodiment will be described. The tomographic image generation unit 42 updates the tomographic image no_f using the following formulas.

$$no\_f(1) \leftarrow no\_f(1) - \beta \times \Delta ef(1)$$

$$no\_f(2) \leftarrow no\_f(2) - \beta \times \Delta ef(2)$$

$$......$$

$$no\_f(num\_pix\_t) \leftarrow no\_f(num\_pix\_t) - \beta \times \Delta ef(num\_pix\_t)$$

$$\text{where } \beta \text{ represents the step size of the update. ... (6)}$$

For a pixel whose value of the tomographic image no_f becomes smaller than the minimum value of 1 of the linear attenuation coefficient numbers as a result of the update, the value of the tomographic image no_f is set to 1. For a pixel whose value of the tomographic image no_f exceeds the maximum value num_f of the linear attenuation coefficient numbers as a result of the update, the value of the tomographic image no_f is set to the maximum value num_f.

As described above, through the process described above, also in the present exemplary embodiment, a high-accuracy tomographic image of the inspection target 60 can be generated regardless of the number of pieces of projection data.

The technique disclosed herein is not limited to the exemplary embodiments described above, and various modifications can be made. For example, the following modifications are possible.

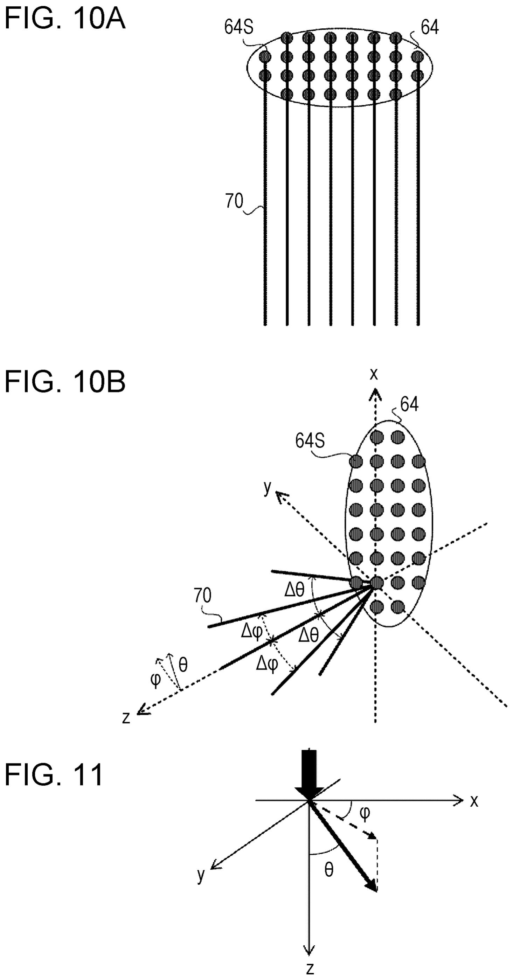

In the exemplary embodiments described above, the scan method is the fan beam method, as an example. Alternatively, the scan method is not limited, and any scan method can be used. For example, the exemplary embodiments described above can also be applied to a pencil beam method. In the pencil beam method, as illustrated in Fig. 10A, a plurality of radiation sources 64S can be set at predetermined equal intervals in a spatially extending predetermined region (the radiation source 64), and paths 70 of the radiation R can be set in parallel from the respective radiation sources 64S. Then, data obtained by a series of parallel scans of the radiation source 64 and the detector 68 is set as one piece of projection data, and data at different rotation angles is set as another piece of projection data. Then, the pieces of projection data are divided into subsets, and the tomographic image generation process described above (see Fig. 7 or Fig. 9) can be performed in a way similar to that for the fan beam method to generate a tomographic image. As illustrated in Fig. 10A, when the paths 70 of the radiation R are set to be parallel, the intensity I0_e(no_e) in Formula (3) above represents, for each path 70, the total sum of the numbers of photons (at the energy e(no_e)) emitted per unit time over a range enclosed by the boundary between the path 70 and an adjacent path 70 and centered on the path 70. Alternatively, also in the pencil beam method, as in the fan beam method, as illustrated in Fig. 5D, the paths 70 may be set from each radiation source 64S at equal intervals with a minute angle. In this case, the intensity I0_e(no_e) in Formula (3) above represents the total sum of the numbers of photons emitted per unit time over a minute angular range centered on each of the paths 70 in Fig. 5D. As described above, in both the fan beam method and the pencil beam method, the radiation R are actually emitted three-dimensionally from the radiation source 64. Thus, to be accurate, the intensity I0_e(no_e) in Formula (3) above represents the total sum of the numbers of photons emitted per unit time over a minute range centered on each of the paths 70 in the beam plane and a minute range of the detector in a direction perpendicular to the beam plane. The pencil beam in the present exemplary embodiment is an example of a parallel beam of the present disclosure.

In the cone beam method, as illustrated in Fig. 10B, a plurality of radiation sources 64S can be set at three-dimensional predetermined intervals in a predetermined region (the radiation source 64) extending in a three-dimensional space, and paths 70 can be set at equal intervals with a minute angle in two straight directions from each of the radiation sources 64S. Specifically, angles in two directions are represented as $\theta$ and $\varphi$, and any direction is represented as a combination ($\theta$, $\varphi$). When the minute angles are represented as $\Delta\theta$ and $\Delta\varphi$, the respective paths 70 can be set in the following directions.

$$..., (-\Delta\theta, -2\times\Delta\varphi/\cos(\Delta\theta)), (-\Delta\theta, -\Delta\varphi/\cos(\Delta\theta)), (-\Delta\theta, 0), (-\Delta\theta, +\Delta\varphi/\cos(\Delta\theta)), (-\Delta\theta, +2\times\Delta\varphi/\cos(\Delta\theta)), ...$$

$$..., (0, -2\times\Delta\varphi), (0, -\Delta\varphi), (0, 0), (0, +\Delta\varphi), (0, +2\times\Delta\varphi), ...$$

$$..., (+\Delta\theta, -2\times\Delta\varphi/\cos(\Delta\theta)), (+\Delta\theta, -\Delta\varphi/\cos(\Delta\theta)), (+\Delta\theta, 0), (+\Delta\theta, +\Delta\varphi/\cos(\Delta\theta)), (+\Delta\theta, +2\times\Delta\varphi/\cos(\Delta\theta)), ...$$

In Fig. 10B, the minute angles $\Delta\theta$ and $\Delta\varphi$ in two directions are indicated by a solid arrow and a wavy arrow, respectively. To equalize the ranges of the minute solid angles centered on the respective paths 70, in the expressions above, the minute angle $\Delta\varphi$ in the angle $\theta$ is corrected to $\Delta\varphi/\cos(\theta)$ (the range of $\theta$ is set to -90 degrees to +90 degrees, and $\Delta\varphi$ is corrected to increase as the absolute value of $\theta$ increases). Then, the pieces of projection data are divided into subsets, and the tomographic image generation process described above (see Fig. 7 or Fig. 9) can be performed in a way similar to that for the fan beam method to generate a tomographic image. In the cone beam method, the detectors 68 are arranged two-dimensionally, and the detector numbers no_d indicate numbers in the two-dimensional arrangement. A tomographic image no_f(no_pix) is a three-dimensional image, and a pixel no_pix in the tomographic image indicates a pixel in the three-dimensional image. Whereas a tomographic image using the fan beam method is constituted by two-dimensional pixels, a tomographic image using the cone beam method is constituted by three-dimensional voxels. In the present exemplary embodiment, not only a two-dimensional image but also a three-dimensional image is referred to as a tomographic image, and three-dimensional voxels are also referred to as pixels. In the case of the cone beam method, the intensity I0_e(no_e) in Formula (3) above represents, for each path 70 in Fig. 10B, the total sum of the numbers of photons (at the energy e(no_e)) emitted per unit time over minute angular ranges $-\Delta\theta/2$ to $+\Delta\theta/2$ and $-\Delta\varphi/\cos(\theta)/2$ to $+\Delta\varphi/\cos(\theta)/2$ in two directions centered on the path 70. Also in both the pencil beam method and the fan beam method, as illustrated in Fig. 10B, the radiation source 64 may be set in a three-dimensional space, and the paths 70 extending in the three-dimensional space may be set (the paths 70 may be set in two directions at intervals with a minute angle). In this case, assuming that each detector is a two-dimensional plane detector and a tomographic image is constituted by three-dimensional voxels, various tables, such as num_path(no_s, no_d) for the number of paths 70 incident on each detector, num_pix(no_s, no_d, no_path) for the number of pixels through which each path 70 passes, no_pix(no_s, no_d, no_path, no_pix_path) for the identification numbers of the pixels, and plen_pix(no_s, no_d, no_path, no_pix_path) for the path lengths, are calculated and generated.

In the exemplary embodiments described above, the paths 70 of the radiation R are set at equal intervals. Alternatively, the intervals may be changed for the respective paths 70 to express a difference in the intensity of the radiation R for the respective paths 70. For example, to set the intensity of the emitted radiation R higher toward the center and lower toward the edges of the radiation source 64, the paths 70 are set at narrower intervals (denser) toward the center and wider intervals (coarser) toward the edges of the radiation source 64. Alternatively, the intensity I0_e(no_e) to be emitted from the radiation source 64 may be changed for each path 70. In this case, in Formula (3) above, the intensity I0_e(no_e) at the energy e(no_e) emitted from the radiation source 64 may be set to the intensity I0_e(no_s, no_d, no_path, no_e) along the path no_path incident on the detector no_d in the scan no_s to change the intensity I0_e according to the path 70. In this case, a table I0_e(no_s, no_d, no_path, no_e) is also generated in the table generation process (see Fig. 4).

Each of the exemplary embodiments described above can also be implemented when the information on the linear attenuation coefficient of the inspection target 60 is represented not by values for two or more energy bands but by a single value. When the information on the linear attenuation coefficient is represented by a single value, the linear attenuation coefficient table f(no_f, no_e) illustrated in Fig. 6 represents a single value f(no_f) for the identification number no_f of each linear attenuation coefficient. Further, Formula (3) above is replaced by Formula (3_2) below.

I = I0 × exp(- {(f(no_f_ext(1)) × plen_pix(no_s, no_d, no_path, 1)) + (f(no_f ext(2)) × plen_pix(no_s, no_d, no_path, 2)) + ... + (f(no_f_ext(num_pix(no_s, no_d, no_path))) × plen_pix(no_s, no_d, no_path, num_pix(no_s, no_d, no_-path)))}),

where

$$\text{no\_f\_ext(no\_pix\_path)} = \text{no\_f(no\_pix(no\_s, no\_d, no\_path, no\_pix\_path))} \ ... \ (3\_2)$$

Then, Formula (2) above is omitted, and the intensities I determined using Formula (3_2) above for the respective paths 70 can be set as I(1), I(2), etc. to determine the virtual projection data g_v from Formula (1) above.

In the exemplary embodiments described above, the paths 70 incident on each detector 68 can be approximately combined into one path. In this case, the tables to be generated in advance are as follows.

· Table num_path(no_s, no_d): represents the number of paths 70 incident on the detector no_d in the scan no_s. Even in a case where the paths 70 are combined into one path, a table of the number of paths 70 before combining is necessary in the first exemplary embodiment.

· Table num_pix(no_s, no_d): represents the number of pixels through which at least one path 70 passes among the paths 1 to num_path(no_s, no_d) incident on the detector no_d in the scan no_s before combining the paths 70 into one path. In a case where the paths 70 are not combined into one path, the table num_pix(no_s, no_d, no_path) is generated, but in a case where the paths 70 are combined into one path, no_path can be omitted.

· Table no_pix(no_s, no_d, no_pix_path): represents the number of the (no_pix_path)-th pixel through which at least one path 70 passes among the paths 1 to num_path(no_s, no_d) incident on the detector no_d in the scan no_s before combining the paths 70 into one path. In a case where the paths 70 are not combined into one path, the table no_pix(no_s, no_d, no_path, no_pix_path) is generated, but in a case where the paths 70 are combined into one path, no_path can be omitted.

· Table plen_pix(no_s, no_d, no_pix_path): represents the path length of one path into which the paths passing through the (no_pix_path)-th pixel through which at least one path 70 passes among the paths 1 to num_path(no_s, no_d) incident on the detector no_d in the scan no_s before combining the paths 70 into one path are combined, that is, the mean value of the path lengths. plen_pix(no_s, no_d, no_pix_path) is determined by extracting all the pixels through which at least one path 70 passes among the paths 1 to num_path(no_s, no_d) before combining the paths 70 into one path, calculating the total sum of the path lengths of the paths 1 to num_path(no_s, no_d) passing through each pixel, and dividing the total sum by num_path(no_s, no_d). The pixel number no_pix_path is common in the tables no_pix(no_s, no_d, no_pix_path) and plen_pix(no_s, no_d, no_pix_path). In a case where the paths 70 are not combined into one path, the table plen_pix(no_s, no_d, no_path, no_pix_path) is generated, but in a case where the paths 70 are combined into one path, no_path can be omitted.

· Table I0_e(no_s, no_d, no_e): represents the total sum of intensities at the energy e(no_e) of the radiation R for all the paths 70 incident on the detector no_d in the scan no_s before combining the paths 70 into one path. In a case where the paths incident on each detector are combined into one path, the number of paths 70 incident on each detector before combining differs between detectors. That is, in Formula (3) above, the intensity I0_e(no_e) at the energy e(no_e) emitted from the radiation source 64 differs for each detector. Thus, the table I0_e(no_s, no_d, no_e) is generated in advance instead of the intensity I0_e(no_e).

· Table plen_pix_sub(no_sub, no_pix): represents the total sum of path lengths through the pixel no_pix for all the paths 70 incident on all the detectors in all the scans belonging to the subset no_sub. Even in a case where the paths 70 are combined into one path, a table of the total sum of the path lengths before combining is necessary in the first exemplary embodiment.

In a case where the paths 70 incident on each detector 68 are approximately combined into one path, Formula (3) above is replaced by Formula (3_3) below.

I_e(no_e) = I0_e(no_s, no_d, no_e) × exp(-{(f(no_f_ext(1), no_e) × plen_pix(no_s, no_d, 1)) + (f(no_f_ext(2), no_e) × plen_pix(no_s, no_d, 2)) + ... + (f(no_f_ext(num_pix(no_s, no_d)), no_e) × plen_pix(no_s, no_d, num_pix(no_s, no_d)))}), where

$$no\_f\_ext(no\_pix\_path) = no\_f(no\_pix(no\_s, no\_d, no\_pix\_path)) \ ... \ (3\_3)$$

Then, the virtual projection data g_v can be determined using Formula (2_3) below instead of Formula (2) above. In this case, Formula (1) above can be omitted.

$$g\_v = (e(1) \times I\_e(1)) + (e(2) \times I\_e(2)) + ... + (e(num\_e) \times I\_e(num\_e)) \ ... \ (2\_3)$$

In step S218 of the tomographic image generation process according to the first exemplary embodiment illustrated in Fig. 7, the formula representing the difference weighted by the path length when the estimated value df(no_s, no_d) of the difference in linear attenuation coefficient number for the path 70 incident on the detector no_d in the scan no_s is weighted by the path length and added to the (no_pix_path)-th pixel no_pix(no_s, no_d, no_pix_path) in the tomographic image df_ave with the mean value of the differences is given by df(no_s, no_d) × plen_pix(no_s, no_d, no_pix_path) × num_path(no_s, no_d).

In a case where the paths 70 incident on each detector 68 are approximately combined into one path and in a case where the information on the linear attenuation coefficient of the inspection target 60 is represented not by values for two or more energy bands but by a single value, the virtual projection data g_v can be generated using Formula (3_4) below instead of Formula (3) above. In this case, Formulas (1) and (2) above can be omitted.

g_v = I0_e(no_s, no_d) × exp(- {(f(no_f_ext(1)) × plen_pix(no_s, no_d, 1)) + (f(no_f ext(2)) × plen_pix(no_s, no_d, 2)) + ... + (f(no_f_ext(num_pix(no_s, no_d))) × plen_pix(no_s, no_d, num_pix(no_s, no_d)))}), where

$$no\_f\_ext(no\_pix\_path) = no\_f(no\_pix(no\_s, no\_d, no\_pix\_path)) \ ... \ (3\_4)$$

In the exemplary embodiments described above, furthermore, the detection efficiencies of the detector 68 at the respective energies are represented as de(1), de(2), ..., and de(num_e), and Formula (2) above may be replaced by Formula (2_5) below to reflect the detection efficiencies of the detector 68 in the intensity I of the radiation R.

I(no_path) = (e(1) × I_e(1) × de(1)) + (e(2) × I_e(2) × de(2)) + ... + (e(num_e) × I_e(num_e) × de(num_e))     (2_5)

In step S212 of the tomographic image generation process according to the first exemplary embodiment described above (see Fig. 7), rf(no_s, no_d) may be calculated as a ratio of linear attenuation coefficient numbers instead of the calculation of the difference df(no_s, no_d) in linear attenuation coefficient number. Specifically, the virtual projection data g_v and the actually measured projection data g(no_s, no_d) are compared. If g_v is larger or smaller, the linear attenuation coefficient numbers of all the pixels through which all the paths incident on the detector no_d in the scan no_s pass are increased or decreased by a predetermined ratio to determine the virtual projection data g_v again, and the determined virtual projection data g_v is compared with the actually measured projection data g(no_s, no_d). This process is repeatedly performed until g_v becomes less than or equal to or greater than or equal to g(no_s, no_d). Then, among g_v immediately before becoming less than or equal to or greater than or equal to g(no_s, no_d) and g_v that becomes less than or equal to or greater than or equal to g(no_s, no_d), the virtual projection data g_v closer to the actually measured projection data g(no_s, no_d) is used, and the ratio of the linear attenuation coefficient numbers during the calculation is stored as rf(no_s, no_d). The increase or decrease by a predetermined ratio means to determine a predetermined increment of the ratio and to perform multiplication while increasing or decreasing the ratio by the increment. For example, this means that a predetermined increment of the ratio is defined as 0.01 and multiplication is performed while increasing the ratio by 1.01 times, 1.02 times, and so on or decreasing the ratio by 0.99 times, 0.98 times, and so on. Since the linear attenuation coefficient number no_f is an integer, it is multiplied by the ratio and then rounded to the nearest integer. To calculate g_v by increasing or decreasing the predetermined amount of the linear attenuation coefficient number by a ratio $\alpha$, where $\alpha$ denotes a predetermined ratio of linear attenuation coefficient numbers, in Formula (3) above, no_f_ext(no_pix_path) is set to round($\alpha \times$ no_f(no_pix(no_s, no_d, no_path, no_pix_path))) instead of no_f(no_pix(no_s, no_d, no_path, no_pix_path)). Note that round(x) means a value obtained by rounding x to the nearest integer. Of course, for a pixel for which round($\alpha \times$ no_f(no_pix(no_s, no_d, no_path, no_pix_path))) exceeds the maximum value of the linear attenuation coefficient numbers (or, conversely, is smaller than the minimum value of 1), the linear attenuation coefficient number is fixed to the maximum value (minimum value of 1).

To calculate the ratio rf(no_s, no_d) of linear attenuation coefficient numbers, in step S218 in the first exemplary embodiment (see Fig. 7), first, rf_ave(no_pix) is initialized to zero for all pixels, where rf_ave(no_pix) denotes a tomographic image with the mean value of ratios. Then, for all the pieces of projection data belonging to the subset j, the ratios rf(no_s, 1) to rf(no_s, num_d) of linear attenuation coefficient numbers are added to the respective pixels of the tomographic image rf_ave with the mean value of ratios while being weighted by the path lengths. Finally, the value of each pixel of rf_ave is divided by the total sum plen_pix_sub of path lengths of the respective pixels to determine a mean value of ratios for the respective pixels, and the tomographic image no_f before the update is multiplied by the ratio and rounded to update the tomographic image. Of course, when the ratio rf(no_s, no_d) of the linear attenuation coefficient numbers is to be added to each pixel of the tomographic image rf_ave, for a pixel whose linear attenuation coefficient number exceeds the maximum value or becomes smaller than the minimum value of 1 as a result of multiplying the tomographic image no_f before the update by the ratio, the ratio is corrected so that the result of the multiplication becomes the maximum value or the minimum value of 1, and then the corrected ratio is added to rf_ave.

In the step of quantizing a tomographic image in each of the exemplary embodiments described above, it is not necessary to quantize all the pixels of the tomographic image. For example, only when a linear attenuation coefficient number falls within a predetermined range close to the value of a member included in the inspection target, the linear attenuation coefficient number may be corrected to the linear attenuation coefficient number of the member. In the example in Fig. 6, the linear attenuation coefficient numbers of the respective pixels are corrected to the linear attenuation coefficient number of air for pixels having linear attenuation coefficient numbers less than or equal to 3, to the linear attenuation coefficient number of aluminum for pixels having linear attenuation coefficient numbers greater than or equal to 4 and less than or equal to 20, and to the linear attenuation coefficient number of iron for pixels having linear attenuation coefficient numbers greater than or equal to 70, and the linear attenuation coefficient numbers of the other pixels need not be corrected. In this case, correction may be performed to always obtain the values of the members included in the inspection target only at the time of the last update (at the time of update when the total update count i is Ni). If the inspection target also includes a member other than the members set in the linear attenuation coefficient table, correction may be performed, also at the time of the last update, only when the linear attenuation coefficient number falls within a predetermined range close to the set members. Further, quantization need not be performed each time the tomographic images for all the subsets are updated, and quantization may be performed each time the update of the tomographic images for all the subsets is repeated a number of times.

As described above, the projection data includes not only direct radiation emitted from the radiation source and attenuated after passing through the inspection target but also scattered radiation generated as new radiation from a portion of the attenuated direct radiation when the direct radiation is attenuated. A large amount of scattered radiation is included particularly in the cone beam method. Thus, in the exemplary embodiments described above, it is preferable to generate a tomographic image by also considering scattered radiation. The probability of occurrence of scattered radiation at each scattering angle is represented by p_s(no_e_in, no_e_out, no_th). no_e_in represents the energy of incident radiation, no_e_out represents the energy of scattered radiation, and no_th represents the angle of the direction of the scattered radiation (hereinafter referred to as the scattering angle) with respect to the direction of the incident radiation (hereinafter referred to as the incident direction). The probability of occurrence p_s represents the likelihood that scattered radiation at the energy no_e_out occurs at the angle no_th with respect to the passage direction when radiation at the energy no_e_in passes a unit travel distance. In the present exemplary embodiment, the energy no_e_in of the incident radiation, the energy no_e_out of the scattered radiation, and the scattering angle no_th are handled discretely and represented as numbers. In Fig. 11, the incident direction is indicated by a thick solid arrow, the direction of the scattered radiation is indicated by a thin solid arrow, the scattering angle is indicated by $\theta$, the direction of the scattered radiation projected onto the plane (xy plane) perpendicular to the incident direction is indicated by a thin wavy arrow, and the angle between the direction of the scattered radiation projected onto the xy plane perpendicular to the incident direction and the x-axis (positive direction) is indicated by $\varphi$. As illustrated in Fig. 11, the scattering angle $\theta$ is the polar angle (zenith angle) in three-dimensional polar coordinates with the z-axis (positive direction) defined as the incident direction, and the angle $\varphi$ is the azimuth angle (declination angle). The probability of occurrence p_s is equally probable for the angle $\varphi$.

When radiation passes through an inspection target, the probability of occurrence of interactions, such as photoelectric effect, coherent scattering (Thomson scattering), incoherent scattering (Compton scattering), and electron pair generation, changes depending on the energy of the radiation. Moreover, the angular distribution of scattered radiation, that is, the probability of scattering at respective angles, varies depending on the type of interaction. In the present exemplary embodiment, accordingly, the total sum of probabilities of occurrence of scattered radiation at the scattering angle no_th due to the respective interactions, which change depending on the energy no_e_in, is represented as p_s(no_e_in, no_e_out, no_th). That is, the total sum of probabilities of occurrence of scattered radiation at each energy no_e_in due to the respective interactions is represented as one function p_s(no_e_in, no_e_out, no_th). Depending on the type of interaction, the energy changes before and after scattering. For example, in the case of coherent scattering, the energy of incident radiation and the energy of scattered radiation are the same, whereas in the case of incoherent scattering, the energy of incident radiation and the energy of scattered radiation are different, and the energy of scattered radiation differs depending on the scattering angle. In the present exemplary embodiment, accordingly, the probability of occurrence p_s of scattered radiation is represented as a function of the energy no_e_in of incident radiation and the energy no_e_out of scattered radiation. Since the probability of occurrence p_s of scattered radiation differs depending on the members included in the inspection target, in the present exemplary embodiment, each member has a probability of occurrence p_s. The probability of occurrence p_s of scattered radiation may be determined by actually measuring the intensity of scattered radiation at each energy no_e_out and each angle no_th for each member, or may be determined theoretically. For example, in the case of incoherent scattering, the probability of scattering at each angle can be calculated using the Klein-Nishina formula. The probability of occurrence p_s of scattered radiation may be given by any means. For example, the probability of occurrence p_s of scattered radiation may be given by a three-dimensional (no_e_in, no_e_out, no_th) table, or may be given by a function having the three variables no_e_in, no_e_out, and no_th as arguments. In the present exemplary embodiment, the total sum of the probabilities of occurrence of scattered radiation due to the respective interactions is represented as one function p_s, but may also be given by a combination of the probability of occurrence of each interaction at each energy no_e_in and the probability of scattering at each energy no_e_out and each angle no_th when each interaction occurs.

Since scattering calculation requires a large amount of computation and takes time, it is preferable that the frequency of the calculation be low in the exemplary embodiments described above. For example, the scattering calculation may be performed after the step of quantizing a tomographic image. Alternatively, the scattering calculation may be performed each time a tomographic image is quantized a predetermined number of times. Since the members included in the inspection target are assigned to the pixels of the tomographic image by the quantization of the tomographic image, each pixel can be assigned the probability of occurrence p_s of scattered radiation for the corresponding member to perform the scattering calculation. In the scattering calculation, pieces of projection data of scattered radiation are generated as g_s(1, 1) to g_s(num_s, num_d), based on the tomographic image. Here, num_s represents the number of scans. Then, the calculated pieces of projection data g_s(1, 1) to g_s(num_s, num_d) of the scattered radiation are subtracted from pieces of actually measured projection data g(1, 1) to g(num_s, num_d) to determine the pieces of projection data of only the direct radiation as g_p(1, 1) to g_p(num_s, num_d). In the next update, the tomographic image is updated based on the pieces of projection data g_p(1, 1) to g_p(num_s, num_d) of only the direct radiation instead of the pieces of actually measured projection data g(1, 1) to g(num_s, num_d). Then, after the updated tomographic image is quantized, the pieces of projection data g_s(1, 1) to g_s(num_s, num_d) of the scattered radiation are generated based on the quantized tomographic image and are subtracted from the pieces of actually measured projection data g(1, 1) to g(num_s, num_d) to generate the pieces of projection data g_p(1, 1) to g_p(num_s, num_d) of only the direct radiation. Then, in the next update, the tomographic image is updated based on the pieces of projection data g_p(1, 1) to g_p(num_s, num_d) of only the direct radiation. This process is repeatedly performed.

A method for calculating scattering based on the probability of occurrence p_s(no_e_in, no_e_out, no_th) of scattered radiation for each pixel to generate pieces of projection data g_s(1, 1) to g_s(num_s, num_d) of scattered radiation will be described. As a method for scattering calculation, when only first scattering (hereinafter referred to as first-order scattering) is calculated and multiple scattering (second-, third-, and higher-order scattering) is not calculated, projection data g_s of scattered radiation can be generated as follows. The focus is placed on the (no_pix_path)-th pixel no_pix(no_s, no_d, no_path, no_pix_path) through which the path no_path incident on the detector no_d in the scan no_s passes. The intensity of scattered radiation generated in the pixel no_pix(no_s, no_d, no_path, no_pix_path) during the attenuation of radiation at the energy no_e along the path no_path is represented as I_pix_out(no_e_out, no_th) and given by Formula (7) below.

$$I\_pix\_out(no\_e\_out,\ no\_th) = I\_pix(no\_e) \times plen\_pix(no\_s,\ no\_d,\ no\_path,\ no\_pix\_path) \times p\_s(no\_e,\ no\_e\_out,\ no\_th) \ ...\ (7)$$

In Formula (7) above, I_pix(no_e) represents the intensity of radiation at the energy no_e along the path no_path at the time of incidence on the pixel no_pix(no_s, no_d, no_path, no_pix_path). In Formula (7) above, I_pix_out(no_e_out, no_th) represents the intensity of scattering of radiation with the incident intensity I_pix(no_e) according to the probability p_s (no_e, no_e_out, no_th) that the radiation is scattered at each scattering angle no_th and each energy no_e_out with respect to the incident direction along the path no_path when the radiation is attenuated after passing along the path no_path a path length plen_pix(no_s, no_d, no_path, no_pix_path). It should be noted that in Formula (7) above, the scattering angle no_th is the polar angle (zenith angle) of the path no_path with respect to the incident direction. In Formula (7), the scattering probability p_s (no_e, no_e_out, no_th) is equally probable for the azimuth angle (declination angle). As a result, the scattering intensity I_pix_out(no_e_out, no_th) has the same intensity for the azimuth angle (declination angle). In the calculation of the scattering intensity I_pix_out(no_e_out, no_th) for incident radiation at the energy no_e using Formula (7), the calculation may be omitted for a combination of no_e_out and no_th for which the scattering probability p_s(no_e, no_e_out, no_th) is zero. After determining the intensity I_pix_out(no_e_out, no_th) of scattered radiation at each energy no_e_out and each scattering angle no_th using Formula (7), it is possible to perform a geometric calculation based on the path no_path in the pixel no_pix(no_s, no_d, no_path, no_pix_path) and the positional relationship between the detectors 1 to num_d in the scan no_s to identify a detector on which each scattered radiation is incident (assuming that each scattered radiation does not undergo multiple scattering (second- and higher-order scattering)) among the detectors 1 to num_d. Similarly, it is possible to perform a geometric calculation to determine the pixels and the path lengths for the pixels through which each scattered radiation passes until reaching the detector after generated at the scattering angle no_th from the path no_path in the pixel no_pix(no_s, no_d, no_path, no_pix_path). Thus, based on the linear attenuation coefficient and the path length at each energy no_e_out in each pixel through which the scattered radiation passes, the intensity when scattered radiation generated at each energy no_e_out and each scattering angle no_th is attenuated and incident on the detector can be determined. In the fan beam method or the cone beam method, scattered radiation generated in the path of direct radiation incident on the detector no_d can be detected by all the detectors 1 to num_d, whereas in the pencil beam method, the scattered radiation can be detected only by the detector no_d. In consideration of the limitation of detectors that can detect the scattered radiation by the scan method, when the detector on which scattered radiation generated in the path of direct radiation incident on the detector no_d is incident is to be identified, a detector from which the scattered radiation can be detected is limited based on the detector number no_d, and then the identification is performed. Through the calculation described above, the intensity at which scattered radiation generated during the attenuation of radiation at the energy no_e along the path no_path is incident on each detector can be determined for the (no_pix_path)-th pixel no_pix(no_s, no_d, no_path, no_pix_path) through which the path no_path incident on the detector no_d in the scan no_s passes. In a similar way, calculation can be performed for all the energies 1 to num_e, calculation can be performed for all the pixels 1 to num_pix(no_s, no_d, no_path), calculation can be performed for all the paths 1 to num_path(no_s, no_d), and calculation can be performed for all the detectors 1 to num_d. Through the calculations described above, the total sum of intensities of scattered radiation incident on the detectors 1 to num_d in the scan no_s can be determined for each of the energies 1 to num_e. By multiplying, for the detectors 1 to num_d in the scan no_s, the intensities of scattered radiation at each energy by each energy and then summing them, the detected intensities, that is, pieces of projection data g_s(no_s, 1) to g_s(no_s, num_d), can be generated (see Formula (2) above). In the summing of the intensities of scattered radiation at each energy, the detection efficiency of the detector may be reflected (see Formula (2_5) above). In a similar way, the pieces of projection data g_s(1, 1) to g_s(num_s, num_d) in all the scans can be generated. A detector on which each scattered radiation generated along the path of direct radiation is incident, the pixels through which each scattered radiation passes until reaching the detector, the path lengths in those pixels, and other variables that are repeatedly used in the scattering calculation and can be determined in advance are preferably determined in advance and tabulated. The pieces of projection data g_s(1, 1) to g_s(num_s, num_d) of only first-order scattered radiation can be generated by the method described above. Alternatively, the pieces of projection data g_s(1, 1) to g_s(1, num_d), g_s(2, 1) to g_s(2, num_d), and so on in the respective scans may be blurred for each scan to virtually generate projection data of multiple-scattered radiation (second-order scattered radiation, third-order scattered radiation, etc.), and the generated projection data may then be added to the original projection data to convert g_s(1, 1) to g_s(num_s, num_d) into projection data of first-order scattered radiation + multiple-scattered radiation. In the pencil beam method, while each piece of data of the detectors 1 to num_d in each scan is data of first-order scattered radiation generated by a different beam, particularly pieces of data generated by beams close to each other are considered as pieces of data of first-order scattered radiation generated by approximately the same beam and are one-dimensionally blurred in a manner similar to that in the fan beam method (such pieces of data are more weakly blurred than in the fan beam method).

A method for performing calculation of multiple scattering as well as first-order scattering based on the probability of occurrence p_s(no_e_in, no_e_out, no_th) of scattered radiation for each pixel to generate pieces of projection data g_s(1, 1) to g_s(num_s, num_d) will be described. In a case where multiple scattering is also included, projection data can be generated by Monte Carlo simulation. Specifically, a large number of photons of radiation at respective energies no_e are emitted from the radiation source 64 in each direction, and it is sequentially determined, for each photon, whether the photon is to travel straight or to be absorbed or scattered each time the photon is advanced a free path length, based on the linear attenuation coefficient and the probability of occurrence p_s(no_e_in, no_e_out, no_th) of scattered radiation for each pixel. If the photon is to be scattered, in which direction the photon is to be scattered is determined. These determinations are repeatedly performed until the photon reaches the detector 68. As a result, the pieces of projection data g_s(1, 1) to g_s(num_s, num_d) can be generated. It should be noted that in the pencil beam method, a detector from which scattered photons can be detected is limited to one detector corresponding to the radiation source 64.

In the exemplary embodiments described above, a region where scans overlap is determined to be a region of a tomographic image, and the attenuation of the radiation R in the remaining region, that is, a region outside the tomographic image, is not considered. The region outside the tomographic image is filled with a substance corresponding to the environment in which the inspection target 60 is present, and the linear attenuation coefficient thereof is known. Further, for each path no_path along which the radiation R emitted from the radiation source 64 in each scan no_s is incident on each detector no_d, the path length from the radiation source 64 to the region of the tomographic image and the path length from the region of the tomographic image to the detector are known. Thus, the virtual projection data g_v can be more accurately determined from these pieces of information. For example, assuming that the region outside the tomographic image is filled with air, the attenuation in the region outside the tomographic image can be calculated based on the linear attenuation coefficient of air to more accurately determine the virtual projection data g_v. As a result, it is possible to more accurately generate a tomographic image of the inspection target. Note that whether or not the attenuation in the region outside the tomographic image is calculated is considered to have a slight effect on the quality of the tomographic image.

In the exemplary embodiments described above, furthermore, the information processing apparatus 10 generates a tomographic image using one piece of projection data at each imaging position. Alternatively, the number of pieces of projection data obtained by the detector 68 at each imaging position is not necessarily one. That is, in the exemplary embodiments described above, the number of pieces of projection data acquired by the acquisition unit 40 of the information processing apparatus 10 for each imaging position so that the tomographic image generation unit 42 of the information processing apparatus 10 generates a tomographic image is one. However, the number of pieces of projection data obtained by the detector 68 at each imaging position is not limited to one and may be two or more. For example, at each imaging position, the inspection target 60 may be irradiated with the radiation R having a plurality of different types of energy from the radiation source 64, and the detector 68 may obtain a plurality of pieces of projection data corresponding to the respective energies. When the radiation R is X-rays, for example, the tube voltage of the radiation source 64 can be changed to change the energy of the X-rays to be emitted. Then, the information processing apparatus 10 may acquire only pieces of projection data corresponding to the respective energies at each imaging position to generate tomographic images corresponding to the respective energies. For example, the inspection target 60 is irradiated with the radiation R having two types of energy and the detector 68 obtains pieces of projection data corresponding to the two types of energy. In this case, the information processing apparatus 10 may acquire only projection data corresponding to the first energy among the pieces of projection data corresponding to the two types of energy at each imaging position to generate a tomographic image corresponding to the first energy, and may similarly acquire only projection data corresponding to the second energy to generate a tomographic image corresponding to the second energy.

In the exemplary embodiments described above, furthermore, the tomographic image generation unit 42 of the information processing apparatus 10 displays a generated tomographic image on the display 26. Alternatively, the tomographic image generation unit 42 may perform only the process of generating a tomographic image and may not necessarily display the tomographic image on the display 26. For example, the tomographic image generation unit 42 may perform only the process of generating tomographic images corresponding to a plurality of types of energy described above, but need not display the respective tomographic images on the display 26. As an alternative to this, the information processing apparatus 10 may combine the tomographic images corresponding to the plurality of types of energy into one to generate a tomographic image (hereinafter referred to as a composite tomographic image) and display the composite tomographic image on the display 26. A composite tomographic image can be generated from tomographic images corresponding to a plurality of types of energy in the following way, for example. For example, as the linear attenuation coefficient number of each pixel in the composite tomographic image, the linear attenuation coefficient numbers of the corresponding pixels in the respective tomographic images corresponding to the plurality of types of energy are compared, and the maximum linear attenuation coefficient number, that is, the linear attenuation coefficient number of the member having the maximum linear attenuation coefficient, is assigned. Alternatively, as the linear attenuation coefficient number of each pixel in the composite tomographic image, a linear attenuation coefficient number that occupies the widest region among the linear attenuation coefficient numbers of the corresponding pixels in the respective tomographic images corresponding to the plurality of types of energy, or specifically, a linear attenuation coefficient number for which the spatial range (areas or volumes) of joining (connected) pixels having the same linear attenuation coefficient number and including the pixel is widest, may be assigned. To generate a composite tomographic image in the way described above, tomographic images corresponding to a plurality of types of energy have common linear attenuation coefficient numbers.

In the exemplary embodiments described above, furthermore, the information processing apparatus 10 generates a tomographic image using one piece of projection data at each of all the imaging positions. Alternatively, the information processing apparatus 10 uses one piece of projection data at each of at least two imaging positions, and need not use one piece of projection data at each of the other imaging positions. That is, a plurality of pieces of projection data (e.g., pieces of projection data corresponding to a plurality of types of energy) may be used depending on the imaging position. In this case, in the exemplary embodiments described above, for an imaging position at which a plurality of pieces of projection data are used, the virtual projection data g_v is determined for each of the plurality of pieces of projection data using Formula (1) above, and the mean value thereof is set as the virtual projection data g_v. Likewise, the mean value of a plurality of pieces of actually measured projection data g(no_s, no_d) is set as the actually measured projection data g(no_s, no_d). To determine the virtual projection data g_v in the way described above, a common linear attenuation coefficient number is used to determine the virtual projection data g_v for each of a plurality of pieces of projection data.

In the exemplary embodiments described above, the hardware structures of processing units that execute various processes, such as the acquisition unit 40 and the tomographic image generation unit 42, may be implemented using various processors described below. As described above, the various processors described above include a CPU that is a general-purpose processor configured to execute software (program) to function as various processing units, and further include a programmable logic device (PLD) that is a processor whose circuit configuration can be changed after manufacturing, such as a field programmable gate array (FPGA), a dedicated electric circuit that is a processor having a circuit configuration designed exclusively for executing specific processing, such as an application specific integrated circuit (ASIC), and so on.

One processing unit may be constituted by one of these various processors, or may be constituted by a combination of two or more processors of the same type or different types (e.g., a combination of a plurality of FPGAs or a combination of a CPU and an FPGA). Alternatively, a plurality of processing units may be configured as a single processor.

Examples of configuring a plurality of processing units by one processor include, first, a form in which, as typified by a computer such as a client and a server, one processor is configured by a combination of one or more CPUs and software and the processor functions as a plurality of processing units. The examples include, second, a form in which, as typified by a system on chip (SoC) or the like, a processor is used in which the functions of the entire system including the plurality of processing units are implemented as one integrated circuit (IC) chip. As described above, the various processing units are configured by using one or more of the various processors described above as a hardware structure.

More specifically, the hardware structure of these various processors may be an electric circuit (circuitry) in which circuit elements such as semiconductor elements are combined.

The exemplary embodiments described above have described an aspect in which the table generation program 30 and the tomographic image generation program 32 are stored (installed) in the storage unit 24 of the information processing apparatus 10 in advance, but this is not meant to be limiting. The table generation program 30 and the tomographic image generation program 32 may be provided on a form recorded on recording media such as a compact disc read only memory (CD-ROM), a digital versatile disc read only memory (DVD-ROM), and a universal serial bus (USB) memory. Alternatively, in an embodiment, each of the table generation program 30 and the tomographic image generation program 32 may be downloaded from an external device via a network.

From the foregoing description, the invention described in the following appendices can be understood.

Appendix 1

An information processing apparatus configured to:

acquire projection data corresponding to each of a plurality of imaging positions at which an inspection target is irradiated with radiation from different directions, such that one piece of projection data is acquired at each of at least two imaging positions among the plurality of imaging positions; and
generate a tomographic image of the inspection target, based on the projection data and attenuation information of inspection target.

Appendix 2

The information processing apparatus according to Appendix 1, wherein
the attenuation information includes attenuation information corresponding to each of at least two members included in the inspection target.

Appendix 3

The information processing apparatus according to Appendix 1 or 2, wherein
the attenuation information includes attenuation information of air.

Appendix 4

The information processing apparatus according to any one of Appendices 1 to 3, wherein
the attenuation information includes attenuation information for at least two energy bands.

Appendix 5

The information processing apparatus according to any one of Appendices 1 to 4, wherein

the processor is configured to
generate the tomographic image, based on scattered radiation produced by the inspection target.

Appendix 6

The information processing apparatus according to any one of Appendices 1 to 5, wherein

the processor is configured to
generate the tomographic image, based on a length of a path along which the radiation is transmitted through the inspection target.

Appendix 7

The information processing apparatus according to any one of Appendices 1 to 6, wherein
the radiation is emitted as any one of a parallel beam, a fan beam, and a cone beam.

Appendix 8

An information processing method including a process performed by a processor, the process including:

acquiring projection data corresponding to each of a plurality of imaging positions at which an inspection target is irradiated with radiation from different directions, such that one piece of projection data is acquired at each of at least two imaging positions among the plurality of imaging positions; and
generating a tomographic image of the inspection target, based on the projection data and attenuation information of the inspection target.

Appendix 9

An information processing program for causing a processor to perform a process including:

acquiring projection data corresponding to each of a plurality of imaging positions at which an inspection target is irradiated with radiation from different directions, such that one piece of projection data is acquired at each of at least two imaging positions among the plurality of imaging positions; and
generating a tomographic image of the inspection target, based on the projection data and attenuation information of the inspection target.

The disclosure of JP2023-008365 filed on January 23, 2023 is incorporated herein by reference in its entirety.
All publications, patent applications, and technical standards described herein are incorporated herein by reference to the same extent as if each individual publication, patent application, or technical standard were specifically and individually indicated to be incorporated by reference.

**Claims**

1. An information processing apparatus comprising:
   at least one processor, the processor being configured to:

   acquire projection data corresponding to each of a plurality of imaging positions at which an inspection target is irradiated with radiation from different directions, such that one piece of projection data is acquired at each of at least two imaging positions among the plurality of imaging positions; and
   generate a tomographic image of the inspection target, based on the projection data and attenuation information of the inspection target.

2. The information processing apparatus according to claim 1, wherein the attenuation information includes attenuation information corresponding to each of at least two members included in inspection target.

3. The information processing apparatus according to claim 1, wherein the attenuation information includes attenuation information of air.

4. The information processing apparatus according to claim 1, wherein the attenuation information includes attenuation information for at least two energy bands.

5. The information processing apparatus according to claim 1, wherein the processor is configured to generate the tomographic image, based on scattered radiation produced by the inspection target.

6. The information processing apparatus according to claim 1, wherein the processor is configured to generate the tomographic image, based on a length of a path along which the radiation is transmitted through the inspection target.

7. The information processing apparatus according to claim 1, wherein the radiation is emitted as any one of a parallel beam, a fan beam, and a cone beam.

8. An information processing method comprising a process performed by a processor, the process comprising:

acquiring projection data corresponding to each of a plurality of imaging positions at which an inspection target is irradiated with radiation from different directions, such that one piece of projection data is acquired at each of at least two imaging positions among the plurality of imaging positions; and
generating a tomographic image of the inspection target, based on the projection data and attenuation information of the inspection target.

9. An information processing program for causing a processor to perform a process comprising:

acquiring projection data corresponding to each of a plurality of imaging positions at which an inspection target is irradiated with radiation from different directions, such that one piece of projection data is acquired at each of at least two imaging positions among the plurality of imaging positions; and
generating a tomographic image of the inspection target, based on the projection data and attenuation information of the inspection target.

FIG. 1

68

60

R

64

10

INFORMATION
PROCESSING
APPARATUS

1

FIG. 2

10

| I/F UNIT 23 | MEMORY 22 | PROCESSOR 20 |

29

| DISPLAY | INPUT DEVICE | STORAGE UNIT |

26

28

24

TABLE GENERATION
PROGRAM — 30

TOMOGRAPHIC IMAGE
GENERATION
PROGRAM — 32

INFORMATION PROCESSING APPARATUS

FIG. 3

10

ACQUISITION UNIT 40

TOMOGRAPHIC IMAGE
GENERATION UNIT 42

INFORMATION PROCESSING APPARATUS

FIG. 4

```
                    ┌─────────────┐
                    │    START    │
                    └──────┬──────┘
                           │
                           ▼
   ┌───────────────────────────────────────────────┐
   │   GENERATE AND STORE PATH LENGTH TABLE         │─── S100
   └───────────────────────┬───────────────────────┘
                           │
                           ▼
   ┌───────────────────────────────────────────────┐
   │ ACQUIRE ATTENUATION INFORMATION AND GENERATE   │─── S102
   │ AND STORE LINEAR ATTENUATION COEFFICIENT TABLE │
   └───────────────────────┬───────────────────────┘
                           │
                           ▼
                    ┌─────────────┐
                    │     END     │
                    └─────────────┘
```

FIG. 5A

FIG. 5B

FIG. 5C

FIG. 5D

FIG. 6

FIG. 7

```
                    ┌─────────────┐
                    │    START    │
                    └──────┬──────┘
                           ↓
        ┌──────────────────────────────────────┐
        │      ACQUIRE PROJECTION DATA          │～ S200
        └──────────────────┬───────────────────┘
                           ↓
        ┌──────────────────────────────────────┐
        │         DIVIDE INTO SUBSETS           │～ S202
        └──────────────────┬───────────────────┘
                           ↓
        ┌──────────────────────────────────────┐
        │   GENERATE INITIAL TOMOGRAPHIC IMAGE  │～ S204
        └──────────────────┬───────────────────┘
                           ↓
        ┌──────────────────────────────────────┐
        │         UPDATE COUNT i = 1            │～ S206
        └──────────────────┬───────────────────┘
                           ↓
        ┌──────────────────────────────────────┐
        │         SUBSET NUMBER j = 1           │～ S208
        └──────────────────┬───────────────────┘
                           ↓
        ┌──────────────────────────────────────┐
        │    PROJECTION DATA NUMBER k = 1       │～ S210
        └──────────────────┬───────────────────┘
                           ↓
        ┌──────────────────────────────────────┐
        │ CALCULATE LINEAR ATTENUATION COEFFICIENT OF │～ S212
        │ TOMOGRAPHIC IMAGE BASED ON PROJECTION DATA  │
        └──────────────────┬───────────────────┘
                           ↓
        ┌──────────────────────────────────────┐
        │              k = k + 1                │～ S214
        └──────────────────┬───────────────────┘
                           ↓
        N            ◇ k > Nk? ◇                 ～ S216
                           ↓ Y
        ┌──────────────────────────────────────┐
        │       UPDATE TOMOGRAPHIC IMAGE        │～ S218
        └──────────────────┬───────────────────┘
                           ↓
        ┌──────────────────────────────────────┐
        │              j = j + 1                │～ S220
        └──────────────────┬───────────────────┘
                           ↓
        N            ◇ j > Nj? ◇                 ～ S222
                           ↓ Y
        ┌──────────────────────────────────────┐
        │       QUANTIZE TOMOGRAPHIC IMAGE      │～ S224
        └──────────────────┬───────────────────┘
                           ↓
        ┌──────────────────────────────────────┐
        │              i = i + 1                │～ S226
        └──────────────────┬───────────────────┘
                           ↓
        N            ◇ i > Ni? ◇                 ～ S228
                           ↓ Y
        ┌──────────────────────────────────────┐
        │       DISPLAY TOMOGRAPHIC IMAGE       │～ S230
        └──────────────────┬───────────────────┘
                           ↓
                    ┌─────────────┐
                    │     END     │
                    └─────────────┘
```

# FIG. 8

| PROJECTION ANGLE | SCAN NUMBER | NUMBER OF SUBSETS: 1 | | NUMBER OF SUBSETS: 2 | | NUMBER OF SUBSETS: 4 | |
|---|---|---|---|---|---|---|---|
| | | SUBSET NUMBER | PROJECTION DATA NUMBER | SUBSET NUMBER | PROJECTION DATA NUMBER | SUBSET NUMBER | PROJECTION DATA NUMBER |
| 0° | 1 | 1 | 1 | 1 | 1 | 1 | 1 |
| 45° | 2 | 1 | 2 | 2 | 1 | 3 | 1 |
| 90° | 3 | 1 | 3 | 1 | 2 | 2 | 1 |
| 135° | 4 | 1 | 4 | 2 | 2 | 4 | 1 |
| 180° | 5 | 1 | 5 | 1 | 3 | 1 | 2 |
| 225° | 6 | 1 | 6 | 2 | 3 | 3 | 2 |
| 270° | 7 | 1 | 7 | 1 | 4 | 2 | 2 |
| 315° | 8 | 1 | 8 | 2 | 4 | 4 | 2 |

# FIG. 9

START

ACQUIRE PROJECTION DATA — S200

DIVIDE INTO SUBSETS — S202

GENERATE INITIAL TOMOGRAPHIC IMAGE — S204

UPDATE COUNT i = 1 — S206

SUBSET NUMBER j = 1 — S208

CALCULATE GRADIENT BASED ON PROJECTION DATA IN SUBSET j — S209

UPDATE TOMOGRAPHIC IMAGE — S218

j = j + 1 — S220

j > Nj? — S222  N / Y

QUANTIZE TOMOGRAPHIC IMAGE — S224

i = i + 1 — S226

i > Ni? — S228  N / Y

DISPLAY TOMOGRAPHIC IMAGE — S230

END

FIG. 10A

FIG. 10B

FIG. 11

# EP 4 657 049 A1

## INTERNATIONAL SEARCH REPORT

| International application No. |
|---|
| **PCT/JP2024/000432** |

### A. CLASSIFICATION OF SUBJECT MATTER

*G01N 23/18*(2018.01)i; *G01N 23/04*(2018.01)i; *G01N 23/046*(2018.01)i; *G01N 23/087*(2018.01)i
FI:   G01N23/18; G01N23/04; G01N23/087; G01N23/046

According to International Patent Classification (IPC) or to both national classification and IPC

### B. FIELDS SEARCHED

Minimum documentation searched (classification system followed by classification symbols)

G01N23/00-23/2276; A61B6/00-6/58

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2014-62743 A (HITACHI, LTD.) 10 April 2014 (2014-04-10) | 1-4, 6-9 |
| | claim 1, paragraphs [0012]-[0037], fig. 1-3, 12-15 | |
| Y | | 5 |
| Y | JP 2013-205267 A (HITACHI-GE NUCLEAR ENERGY, LTD.) 07 October 2013 (2013-10-07) | 5 |
| | claim 1, paragraph [0009] | |
| A | JP 2019-58480 A (HAMAMATSU PHOTONICS K.K.) 18 April 2019 (2019-04-18) | 1-9 |
| | entire text, all drawings | |
| A | JP 2005-95397 A (GE MEDICAL SYSTEMS GLOBAL TECHNOLOGY CO. LLC) 14 April 2005 (2005-04-14) | 1-9 |
| | entire text, all drawings | |
| A | WO 2015/064446 A1 (HITACHI MEDICAL CORP.) 07 May 2015 (2015-05-07) | 1-9 |
| | entire text, all drawings | |

☑ Further documents are listed in the continuation of Box C.          ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 March 2024** | **19 March 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

EP 4 657 049 A1

**INTERNATIONAL SEARCH REPORT**

International application No.

**PCT/JP2024/000432**

| C. | DOCUMENTS CONSIDERED TO BE RELEVANT | |
|---|---|---|
| Category* | Citation of document, with indication, where appropriate, of the relevant passages | Relevant to claim No. |
| A | US 2017/0023496 A1 (PRISMATIC SENSORS AB) 26 January 2017 (2017-01-26) entire text, all drawings | 1-9 |

Form PCT/ISA/210 (second sheet) (July 2022)

32

**INTERNATIONAL SEARCH REPORT**
Information on patent family members

International application No.

**PCT/JP2024/000432**

| Patent document cited in search report | | | Publication date (day/month/year) | Patent family member(s) | | | Publication date (day/month/year) |
|---|---|---|---|---|---|---|---|
| JP | 2014-62743 | A | 10 April 2014 | US | 2014/0079180 | A1 | |
| | | | | claim 1, pars. paragraphs [0027]-[0050], fig. 1-3, 9-12 | | | |
| | | | | EP | 2711695 | A1 | |
| JP | 2013-205267 | A | 07 October 2013 | (Family: none) | | | |
| JP | 2019-58480 | A | 18 April 2019 | (Family: none) | | | |
| JP | 2005-95397 | A | 14 April 2005 | (Family: none) | | | |
| WO | 2015/064446 | A1 | 07 May 2015 | US | 2016/0199018 | A1 | |
| | | | | CN | 105451659 | A | |
| US | 2017/0023496 | A1 | 26 January 2017 | WO | 2015/156711 | A1 | |
| | | | | EP | 3129957 | A1 | |
| | | | | CN | 106233335 | A | |

Form PCT/ISA/210 (patent family annex) (July 2022)

**EP 4 657 049 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014061274 A

- JP 2023008365 A